# EUROPEAN PATENT APPLICATION

(11) **EP 3 900 741 A1**
(43) Date of publication of application: **27.10.2021**
(21) Application number: 20739028.7
(22) Date of filing: 07.01.2020
(51) Int. Cl.: A61K 39/245, A61P 31/22, C12N 15/861

(54) **SAMRNA VACCINE AND PREPARATION METHOD THEREFOR**

(30) Priority: 07.01.2019 CN 201910010764
(71) Applicant: Cansino Biologics Inc., Tianjin 300457 (CN)
(72) Inventor: ZHU, Tao, Tianjin 300457 (CN); LI, Junqiang, Tianjin 300457 (CN); CHAO, Shoubai, Tianjin 300457 (CN); XIN, Chunlin, Tianjin 300457 (CN); MIAO, Wei, Tianjin 300457 (CN); LU, Xishan, Tianjin 300457 (CN)
(74) Representative: Hanna Moore + Curley
(86) International application number: PCT/CN2020/070734
(87) International publication number: WO 2020/143634

(57) **Abstract**

Disclosed is an SamRNA vaccine, including a recombinant viral vector which includes: i) a viral gene replication complex including nucleotide sequences encoding viral gene replication-related proteins nsP1, nsP2, nsP3, and nsP4; and ii) a nucleotide sequence encoding at least one antigen. According to the SamRNA vaccine of the present invention, in addition to that a promoter of a modified adenoviral vector itself can transcribe an antigen gene to form mRNA, the viral gene replication-related proteins nsP1-4 use RNA as a template to synthesize a large amount of mRNAs, and the immune effect of a target antigen is greatly improved.

## Description

### TECHNICAL FIELD

The present invention belongs to the technical field of vaccines, and particularly relates to a process technology for producing an mRNA vaccine by a modified adenoviral vector and a preparation method thereof, and the mRNA vaccine produced by the adenoviral vector can be continuously amplified by using RNA as a template to form more mRNAs, and is called samRNA (self-amplifying mRNA) for short.

### BACKGROUND

An mRNA vaccine is a gene vaccine with immunity, safety and flexibility. The mRNA vaccine can stimulate an immune system to produce balanced and long-term protection. Some mRNA vaccines themselves have the characteristics of a vaccine adjuvant. The mRNA vaccines can stimulate the immune system in many manners such as generating multiple cytokines, so as to enhance the response ability of an immune body, shorten the immune response time and increase the ability of antibody synthesis and release.

In 1990, scientists injected messenger RNA (mRNA) transcribed *in vitro* into mice, and found that the mRNA could express its activity in the mice, produce related proteins, and have dose-dependence by detection. This method of injecting the mRNA directly can produce an immune response by expressing a specific protein, which is an embryonic form of an mRNA therapy.

Compared with traditional vaccines, the mRNA has more advantages in safety, causes no insertion of gene mutation, and can be degraded by normal cells, and its half-life can be changed by adjusting sequence modification and delivery vectors. More importantly, traditional vaccines are powerless against many new viruses, not to mention cancer, which is a disease which seriously threatens human health. The acting mechanism of the mRNA makes it like a meal menu. As long as the RNA sequences are encoded, cells can be turned into small drug factories, and the mRNA guides the cells to produce specific proteins to exert a systemic pharmaceutical effect by themselves.

An acting mechanism of the mRNA vaccine: the mRNA participates in the intermediate steps of DNA transcription and protein generation. Currently, there are two kinds of RNAs used for making vaccines, i.e., a non-replicating mRNA and a self-amplifying mRNA (SamRNA). A antigen encoded by a traditional mRNA vaccine contains 5' and 3' untranslated regions (UTRs), while the self-amplifying mRNA can not only encode an antigen, but also have a sequence similar to a virus replication process, so that it can replicate in cells and increase the expression quantity of the protein.

The mRNA can be formed by transcribing through a cDNA template *in vitro,* and an open reading frame (ORF) for protein encoding is added into the mRNA in the late transcription period, so that the synthesized mRNA has the function of encoding a protein. The ORF consists of at least two important elements: a "cap" structure at the 5' terminal and a "tail" of poly A. In addition, untranslated regions (UTRs) and other complexes are increased to help the stable transcription of the mRNA.

However, a naked mRNA will be degraded when entering the body directly, and efficient mRNA delivery is a guarantee for vaccine efficacy, so developing an efficient mRNA delivery vector is a key factor to ensure vaccine effectiveness. Currently, the administration modes of the mRNA are mainly intradermal or intraarticular injection. However, for such an injection mode, due to the degradation of the mRNA *in vivo,* administration with a large dosage is needed to stimulate the body to produce an effective immune response. It has been reported in literatures that protamine and other polymers, such as liposomes, can be used for encapsulating the mRNA, thereby avoiding a large amount of damages to the mRNA by proteases in the body. Compared with direct injection, this encapsulation method can effectively increase the body absorption of antigens, but there are still some defects to be overcome, such as the effective encapsulation amount, the release time of the encapsulated mRNA *in vivo,* and the release and transfer of the mRNA *in vivo.*

Currently, an adenovirus (Ad) vector system has been widely used in the development of gene therapy drugs and vaccines, there are also many reports of the Ad vector system in clinic, and its safety and reliability as a gene delivery vector have been fully proved. According to whether it can replicate, the adenoviral vector is divided into a replication type and a replication-deficient type. Currently, the replication-defective-type adenoviral vector is more commonly used. Compared with the replication-type adenoviral vector, the replication-defective-type adenoviral vector has better safety and lower immunogenicity of the vector itself. An adenoviral vector vaccine refers to that a protective antigen gene is recombined into a virus genome with an adenovirus as a vector. The antigen gene in the adenoviral vector is protected by a virus capsid protein, which can avoid the degradation of the carried gene by various proteases in the host and effectively solve the degradation risk of the mRNA from an injection site to a host cell. After the host cell is infected with the adenoviral vector, the antigen gene or target gene carried by the adenoviral vector can be expressed into a protein.

Besides being easy to degrade and low in bioavailability, the mRNA vaccine also has a problem of copy number. Whether the mRNA is directly injected or a commonly-used protamine-encapsulated mRNA, one mRNA can only be effectively translated once in an organism, which greatly limits the availability of the mRNA as the vaccine. Therefore, in order to solve this phenomenon, it is necessary to design a reproducible system to ensure that the mRNA can be continuously produced in the host cell. After the host cell is infected with the adenoviral vector, the carried genes are injected into the host cell, and these genes can synthesize mRNAs by utilizing respective base pairs of the host cell. More importantly, the adenovirus is a DNA virus, and the gene amplification ability of the DNA virus in the host is much higher than that of a RNA virus. Therefore, using the adenovirus as a vector will greatly increase the copy number of the mRNA.

After the host cell is infected with the adenoviral vector vaccine, the copy number of the target gene is determined by the strength of the promoter of the adenoviral vector itself, and especially for the replication-defective-type adenoviral vector, its replication function is still relatively weak. In order to solve this problem, in the present invention, the inventor modifies the adenovirus gene to realize the combined action of the promoter of the adenoviral vector itself and the modifying gene, thereby greatly improving the expression rate of the target antigen. The DNA of an adjuvant can also be added into the adenoviral vector carrying the mRNA gene. The adenoviral vector system constructed in this way can not only improve the immunogenicity of the body, but also produce the effect of the adjuvant.

### SUMMARY

The present invention provides an SamRNA vaccine, including a recombinant viral vector which includes: i) a viral gene replication complex including nucleotide sequences encoding viral gene replication-related proteins nsP1, nsP2, nsP3 and nsP4; and ii) a nucleotide sequence encoding at least one antigen.

Preferably, the SamRNA vaccine further includes: iii) a promoter for transcribing an antigen gene.

Preferably, the recombinant viral vector is a recombinant adenovirus, a chimpanzee adenovirus, a recombinant vesicular stomatitis virus, a recombinant poxvirus, a recombinant dengue virus, a recombinant Kunjin virus, a recombinant sendai virus, or a recombinant canine distemper virus. More preferably, the recombinant viral vector is a recombinant adenoviral vector and a chimpanzee adenoviral vector, and more preferably, the adenovirus can be any one of Ad1-Ad52.

The antigen of the present invention causes an immune response against bacteria, viruses, fungi or parasites.

In one embodiment of the present invention, the antigen is a human herpes zoster virus gE protein, a rotavirus VP4 or VP7, an HPV-L1 protein, or a Ebola virus gP protein.

The antigen of the present invention can also be a tumor-specific antigen. For example, the tumor-specific antigen is selected from NY-ESO-1, SSX2, SCP1, RAGE, BAGE, GAGE, MAGE family polypeptides, p53, p21/Ras, CDK4, MUM1, caspase-8, CIA0205, HLA-A2-R1701, β-catenin, TCR, BCR-abl, triosephosphate isomerase, KIA0205, CDC-27, LDLR-FUT, Galectin 4, Galectin 9, protease 3, WT 1, carbonic anhydrase, aldolase A, PRAME, HER-2/neu, mammaglobin, alpha-fetal protein, KSA, gastrin, telomerase catalytic protein, MUC-1, G-250, p53, carcino-embryonic antigens, melanoma-melanocyte differentiation antigens, PAP, PSA, PSMA, PSH-P1, PSM-P1, PSM-P2, p15, Hom/Mel-40, H-Ras, E2A-PRL, H4-RET, IGH-IGK, MYL-RAR, EB viral antigens, EBNA, human papillomavirus antigens, hepatitis B and C viral antigens, human T-lymphotropic viral antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791Tgp72, β-HCG, BCA225, BTAA, CA 125, CA 15-3(CA 27.29\BCAA), CA 195, CA 242, CA-50, CAM43, CD68\KP1, CO-029, FGF-5, Ga733(EpCAM), HTgp-175, M344, MA-50, MG7-Ag, MOV18, NB/70K, NY-CO-1, RCAS1, SDCCAG16, TAAL6, TAG72, TLP, and TPS.

Preferably, the recombinant viral vector is obtained by co-transfecting a modified adenoviral skeleton plasmid and a shuttle plasmid containing an antigen gene. The modified adenoviral skeleton plasmid includes a viral gene replication complex, which is derived from proteins related to replication of coding virus genes of RNA viruses, and more preferably, the viral gene replication complex is nucleotide sequences of nsP1, nsP2, nsP3 and nsP4 from alphaviruses. The adenoviral skeleton plasmid is selected from pAdEasy-1, pAdEasy-2, pBHG11, pBHG-fiber5, or pBHG-fiber35.

In one embodiment of the present invention, the recombinant viral vector further includes: iv) a nucleotide sequence encoding at least one adjuvant. For example, the nucleotide sequence encoding the at least one adjuvant is selected from GM-CSF, IL-17, IFNg, IL-15, IL-21, anti-PD1/2, lactoferrin, protamine, IL-1, IL-2, IL-3, IL-4, IL-5, IL-6, IL-7, IL-8, IL-9, IL-10, IL-12, INF-α, INF-γ, Lymphotoxin-a, and hGH. The present invention provides a method for preparing an SamRNA vaccine, including the steps of: constructing a modified adenoviral skeleton plasmid; cloning an antigen gene fragment; constructing a shuttle plasmid; and co-transfecting and packaging the shuttle plasmid and the adenoviral skeleton plasmid.

Preferably, the step of constructing the modified adenoviral skeleton plasmid includes cloning the viral gene replication complex and the promoter, and connecting the viral gene replication complex and the promoter with the adenoviral skeleton plasmid. Preferably, the adenoviral skeleton plasmid includes a viral gene replication complex, which is gene sequences encoding viral gene replication-related proteins nsP1, nsP2, nsP3 and nsP4. The amino acid sequences of the viral gene replication-related proteins nsP1, nsP2, nsP3 and nsP4 are shown in SEQ ID NO.1, SEQ ID NO.2, SEQ ID NO.3 and SEQ ID NO.4.

Preferably, the adenoviral skeleton plasmid further includes a promoter which is the promoter for transcribing an antigen gene, and a sequence of the promoter is different due to the change of the nucleotide sequence encoding at least one antigen. Preferably, in the present invention, a gene sequence of the promoter is as shown in SEQ ID NO.5.

The adenoviral skeleton plasmid is selected from pAdEasy-1, pAdEasy-2, pBHG11, pBHG-fiber5, pBHG-fiber35, etc.

Preferably, a construction method of a shuttle plasmid containing an antigen gene adopts a conventional experimental method in the industry, which is generally adopted in the art, for example, a nucleotide sequence encoding at least one antigen is synthesized, wherein the same enzymes as those used for the shuttle plasmid are designed to be introduced at both terminals of the synthesized nucleotide sequence encoding at least one antigen, the target antigen and the shuttle plasmid are subjected to double enzyme digestion respectively, then the nucleotide fragments are recovered, and the nucleotide sequence encoding the at least one antigen is connected with the plasmid through a T4 DNA ligase. Specific reference can be made to the method of introducing the viral gene replication complex and the promoter into the skeleton plasmid.

According to the method for preparing the SamRNA vaccine provided by the present invention, a SamRNA vaccine capable of preventing this disease can be developed as long as any antigen gene (i.e., the nucleotide sequence encoding at least one antigen) is inserted into the shuttle plasmid. The vaccine is obtained by co-transfection and recombination of the aforementioned adenoviral skeleton plasmid in which the genes of the viral gene replication complex and the promoter are inserted and the shuttle plasmid containing the antigen gene. The vaccine includes the recombinant viral vector, which includes: i) a viral gene replication complex, which includes nucleotide sequences encoding viral gene replication-related proteins nsP1, nsP2, nsP3 and nsP4, and ii) a nucleotide sequence encoding at least one antigen.

Preferably, the antigen gene is a DNA sequence that can encode any antigen, and any antigen gene that has been discovered or publicly reported can be recombined into an adenoviral vector through the shuttle plasmid, and after the skeleton plasmid and the shuttle plasmid are co-transfected, the SamRNA vaccine of the present invention can be obtained.

Preferably, the antigen gene is selected from a DNA sequence encoding a human herpes zoster virus gE protein, a DNA sequence of a rotavirus VP4 or VP7, a DNA sequence of an HPV-L1 protein, and a DNA sequence of an Ebola virus gP protein, etc.

The shuttle vector is selected from pDC311, pDC312, pDC315, pDC316, p-Shuttle, p-Shuttle-CMV, pAdTrack, pAdTrack-CMV, etc.

The present invention provides a pharmaceutical composition including the SamRNA vaccine, and a pharmaceutically-acceptable adjuvant.

The pharmaceutically-acceptable adjuvant includes a diluent, a solubilizer, an adhesive, a lubricant, a suspending agent, etc.

The dosage form of the pharmaceutical composition includes but is not limited to a freeze-dried preparation, liquid preparation, an emulsion, etc.

Preferably, the pharmaceutical composition is a freeze-dried preparation, and the auxiliary materials of the freeze-dried preparation include mannitol, sucrose, human albumin, and a PB buffer (for maintaining pH of the preparation). In an embodiment of the present invention, in the pharmaceutical composition, the concentration of the mannitol is 10-500 mg/ml, the concentration of the sucrose is 10-500 mg/ml, the concentration of the human albumin is 25-100 mg/ml, and the concentration of the PB buffer is 1-100 mM.

Preferably, the SamRNA vaccine is a liquid injection, and the auxiliary materials added in the liquid preparation include human albumin and the PB buffer. The concentration of the human albumin is 25-100 mg/ml, and the concentration of the PB buffer is 1-100 mM.

The present invention provides a modified adenoviral skeleton plasmid in which a gene of a viral gene replication complex is inserted, wherein the viral gene replication complex is gene sequences encoding viral gene replication-related proteins nsP1, nsP2, nsP3 and nsP4. The gene sequences are inserted into the adenoviral skeleton plasmid in an nspl--nsp2--nsp3--nsp4 connection manner, and one or more linkers are added between two adjacent protein genes. The linkers include but are not limited to G4S, an LE linker 1 (with a sequence of TTAGAA), an LE linker 2 (with a sequence of CTCGAA), or DEL (with a sequence of GATGAACTG). The amino acid sequences encoding the viral gene replication-related proteins nsP1, nsP2, nsP3 and nsP4 are SEQ ID NO.1, SEQ ID NO.2, SEQ ID NO.3 and SEQ ID NO.4, respectively. Preferably, the adenoviral skeleton plasmid further includes a promoter which is a promoter for transcribing an antigen gene, and a sequence of the promoter varies with the change of the antigen gene; the promoter can be derived from a promoter when a non-structural protein of a RNA virus is replicated, and can also be derived from common promoters on various expression vectors. Preferably, in the present invention, a gene sequence of the promoter is as shown in SEQ ID NO.5.

Preferably, the promoter and the replication complex genes on the adenoviral skeleton plasmid are codon optimized for a purpose of improving the expression of the replication complex in a host cell, thereby promoting transcription to form more copies of the mRNA. The amino acid sequences of nsP1, nsP2, nsP3 and nsP4 are SEQ ID NO.1, SEQ ID NO.2, SEQ ID NO.3 and SEQ ID NO.4, respectively. The gene sequence of the promoter is as shown in SEQ ID NO.5.

The sequences of nsP1, nsP2, nsP3, nsP4 and the promoter adopt a gene synthesis method, which is known to those of ordinary skills in the art. nsP1, nsP2, nsP3 and nsP4 are directly synthesized into one fragment, and one or more linkers are added between adjacent genes. Enzymatic cleavage sites are added to both terminals of the synthesized sequence, so that the gene fragment can be inserted into the adenoviral skeleton plasmid simply by a T4 DNA ligase.

The adenoviral skeleton plasmid is selected from pAdEasy-1, pAdEasy-2, pBHG11, pBHG-fiber5, and pBHG-fiber35.

The present invention provides a method for preparing the modified adenoviral skeleton plasmid, including the steps of:
cloning the genes of the viral gene replication complex and the promoter, wherein the amino acid sequences of nsP1, nsP2, nsP3 and nsP4 are SEQ ID NO.1, SEQ ID NO.2, SEQ ID NO.3 and SEQ ID NO.4, respectively. The gene sequence of the promoter is as shown in SEQ ID NO.5.

The conventional technology mastered in this art can provide the synthesis business of this gene; and gene synthesis is conducted by adopting conventional experimental techniques in the art. Enzymatic cleavage sites are added to both terminals of the synthesized promoter gene fragment.

Preferably, the amino acid sequences of the viral gene replication complexes nsP1, nsP2, nsP3 and nsP4 are SEQ ID NO.1, SEQ ID NO.2, SEQ ID NO.3 and SEQ ID NO.4. The four protein genes are synthesized into one gene fragment by adopting the order of nsP1-linker--nsP2-linker--nsP3-linker--nsP4, and one or more linkers are added between two protein genes. The linkers include but are not limited to G4S, an LE linker 1 (with a nucleotide sequence of TTAGAA), an LE linker 2 (with a nucleotide sequence of CTCGAA), or DEL (with a nucleotide sequence of GATGAACTG). Enzymatic cleavage sites are added to both sides of the gene fragment.

Insertion of the gene fragments of the viral gene replication complex and the promoter into the adenoviral skeleton plasmid: the connection of the gene fragments of the viral gene replication complex and the promoter with the adenoviral skeleton plasmid is conducted by adopting a conventional experimental method in the industry, and those of ordinary skills in molecular biology in the art can carry out this operation.

Preferably, the gene fragment of the viral gene replication complex is connected with the adenoviral skeleton plasmid, and the specific operation is that the adenoviral skeleton plasmid and the viral gene replication complex are respectively subjected to double enzyme digestion at 37°C; after the enzyme digestion is completed, a target gene fragment is recovered through gel extraction, the recovery of the target gene fragment is conducted by adopting a gel extraction kit, and the specific operation can be carried out according to the instructions of the kit. Then, the gene fragment of the viral gene replication complex and the adenoviral skeleton plasmid which are subjected to double enzyme digestion are ligated by a DNA ligation kit/T4DNA ligase, and the ligation is conducted overnight. Thereafter, the ligation product is transformed into DH5a competent cells. An LB medium is inoculated with clones identified as positive by PCR, and the clones are kept at 37°C at 200-2250 rpm overnight. On the next day, the bacterial cells are recovered, and the plasmid is recovered by a plasmid extraction kit.

Preferably, the gene fragment of the promoter is inserted into the adenoviral skeleton plasmid to which the fragment of the viral gene replication complex has already been ligated. The method for inserting the gene fragment of the promoter into the adenoviral skeleton plasmid is similar to the aforementioned method. The adenoviral skeleton plasmid to which the viral gene replication complex has already been ligated, and the promoter, are subjected to double enzyme digestion at 37°C; after the enzyme digestion is completed, a target gene fragment is recovered through gel extraction, the recovery of the target gene fragment is conducted by adopting a gel extraction kit, and the specific operation can be carried out according to the instructions of the kit. Then, the gene fragment of the viral gene replication complex and the adenoviral skeleton plasmid which are subjected to double enzyme digestion are ligated by a DNA ligation kit/T4DNA ligase, and the ligation is conducted overnight. Thereafter, the ligation product is transformed into DH5a competent cells. An LB medium is inoculated with clones identified as positive by PCR, and the clones are kept at 37°C at 200-2250 rpm overnight. On the next day, the bacterial cells are recovered, and the plasmid is recovered by a plasmid extraction kit.

Preferably, the constructed adenoviral skeleton plasmid is identified by double enzyme digestion and PCR methods.

The present invention solves the risk that the mRNA vaccine is greatly degraded in vivo; although the existing encapsulating technology can avoid the significant degradation of the mRNA vaccine, the defect that it is difficult for the mRNA to directly enter cells (low bioavailability) cannot be solved. The samRNA vaccine of the present invention is an adenoviral vector carrying an antigen, and the adenoviral vector can directly infect human and animal cells, thereby injecting the target antigen gene into the host cell. The conventional mRNA vaccine can only be translated once after entering the host cell, while the samRNA vaccine of the present invention can use RNA as a template to synthesize a large amount of mRNAs, thereby greatly increasing the expression quantity of the antigen.

A common replication-defective-type adenoviral vector can only replicate in cells like HEK293, and can only infect and cannot replicate for humans and animals, so its replication ability in organisms is limited. However, after the adenoviral vector vaccine in which the viral gene replication complex and the promoter are inserted, provided by the present invention, infects the host cell, nucleic acid is injected into the host cell. Then a series of dynamic reactions will take place in the host cell. Firstly, the transcription of the gene of the viral gene replication complex and the translation of a protein occur. Meanwhile, the antigen gene is transcribed into mRNA. With the translation of the proteins nsP1, nsP2, nsP3 and nsP4, the four proteins will form a viral gene replication complex of a viral genome. Then, under the action of the viral gene replication complex, a large number of mRNAs are synthesized by taking an antigen gene RNA as a template, and these mRNAs use various proteases of the host cell to synthesize the target antigens. The process in which the viral gene replication complex takes the RNA as a template to synthesize mRNA, is a continuous process, and antigen expression lasts longer, so with the modified adenoviral vector, the action of the SamRNA vaccine lasts longer.

The literature reports that a virus is used as a vector of SamRNA, but the shortcomings are obvious. First of all, the viruses used for samRNA are all RNA viruses, and are positive-sense RNA viruses, and these viruses have limited infection and replication abilities. Host restriction makes it very difficult to produce SamRNA using the virus as the vector, and strict host cell restriction makes it difficult for a conventional production process to meet the production of such a virus. In addition, the limited amplification capacity further seriously affects the yield of the virus. In addition, for the SamRNA using the virus as the vector, the outer-membrane structure gene of the RNA virus is replaced by the antigen gene in the process of genome recombination and construction. The deletion of the outer-membrane structure gene of the virus has the advantage that no virus will be assembled after animal and human host cells are infected with the virus, thereby reducing the risk of infection or pathopoiesia. However, the shortcomings are obvious. The depletion of the structural gene causes the virus vector vaccine to be unable to complete the assembly of virus particles under conventional conditions, and the technical difficulty of commercial production is relatively high.

### BRIEF DESCRIPTION OF THE DRAWINGS

FIG. 1 shows the identification results of inserting a viral gene replication complex protein gene into a skeleton plasmid;
FIG. 2 shows the identification results of inserting a promoter sequence into the skeleton plasmid;
FIG. 3 shows a modified skeleton plasmid;
FIG. 4 shows a flow diagram of constructing Ad-SamRNA-gE;
FIG. 5 shows the double enzyme digestion verification of Ad-SamRNA-gE;
FIG. 6 shows a chromatogram of Ad-SamRNA-gE purified by CL-4B;
FIG. 7 shows the purity analysis of Ad-SamRNA-gE;
FIG. 8 shows the immunogenicity of different vectors, wherein the left side is the immunogenicity of primary immunization, and the right side is the immunogenicity of secondary immunization;
FIG. 9 shows the expression quantities of gE genes of different vectors; and
FIG. 10 shows that the molecular adjuvant enhances the immunogenicity of Ad-SamRNA-gE, wherein the left side is the immunogenicity of primary immunization, and the right side is the immunogenicity of secondary immunization.

### DETAILED DESCRIPTION

The technical solutions in the embodiments of the present invention will be clearly and completely described below. Apparently, the described embodiments are merely a part of embodiments rather than all embodiments of the present invention. All other embodiments obtained by a person of ordinary skill in the art based on the embodiments of the present invention without creative efforts shall fall within the protection scope of the present invention.

### Embodiment 1 insertion of viral gene replication complex and promoter gene fragments into a skeleton plasmid

Synthesis of viral gene replication complex and promoter gene fragments:
In a specific implementation, the gene sequences of the viral gene replication complexes nsP1, nsP2, nsP3 and nsP4 were SEQ ID NO.6, SEQ ID NO.7, SEQ ID NO.8 and SEQ ID NO.9, respectively, and the viral gene replication complex gene fragment is synthesized according to the aforementioned sequences, wherein four antigen protein genes were synthesized into a large gene fragment.

For the promoter, a gene fragment of the promoter was synthesized according to SEQ ID NO.5, and meanwhile, enzymatic cleavage sites were added at both terminals of the gene fragment of the promoter.

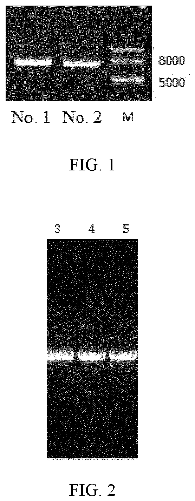

Taking a pAdEasy-1 adenoviral skeleton plasmid as an example, a process of inserting the viral gene replication complex and the promoter into pAdEasy-1 was presented. Firstly, the gene fragment of the viral gene replication complex was inserted into the pAdEasy-1 adenoviral skeleton plasmid, wherein the 4 synthesized long protein gene fragments of the viral gene replication complex contained pacI enzymatic cleavage sites on both sides. pAdEasy-1 and the 4 protein genes of the viral gene replication complex were respectively digested with a pac I enzyme. After gel extraction, ligation was conducted by a T4 DNA ligase. Theoretically, there were two ligation manners of the protein genes of the viral gene replication complex (insertion into the skeleton plasmid in an inverted order and insertion into the skeleton plasmid in a sequential order). After the ligation product was transformed into DH5a, monoclones were selected, and the vector in which the genes were inserted into the skeleton plasmid as expected was identified by a PCR method. The results of PCR identification were shown in FIG. 1. The clone No.1 and the clone No.2 were both correctly inserted into the protein gene fragment of the viral gene replication complex, and the clone No. 1 was selected to be subjected to an operation of inserting the gene fragment of the promoter.

The gene of the promoter and the skeleton plasmid into which the gene of the viral gene replication complex has been inserted, were digested with ClaI, and then ligated with a T4 DNA ligase after gel extraction. Theoretically, there were two ligation manners of the protein genes of the promoter (insertion into the skeleton plasmid in an inverted order and insertion into the skeleton plasmid in a sequential order). After the ligation product was transformed into DH5a, monoclones were selected, and the vector in which the genes were inserted into the skeleton plasmid as expected was identified by a PCR method. The results of PCR identification were shown in FIG. 2. In this step of operation, clones No.3, NO.4 and NO.5 were correctly inserted into the promoter sequence.

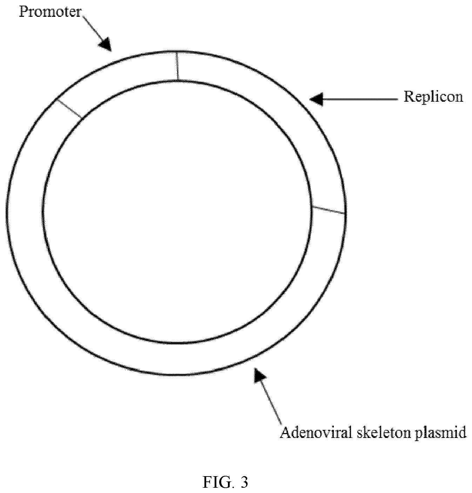

After the aforementioned two steps, the skeleton plasmids pAdEasy-No. 13, No.4 and No.5 with addition of the sequences of the viral gene replication complex and the promoter, will be obtained. The positions of the promoter and the viral gene replication complex in the pAdEasy-1 skeleton plasmid were shown in FIG. 3. According to the same method, the insertion of sequences of the promoter and the viral gene replication complex could be completed on other skeleton plasmids.

### Embodiment 2 construction of Ad-SamRNA-gE

Taking the herpes zoster gE protein antigen as an example, the display of a construction process of an Ad (adenovirus)-SamRNA vaccine was conducted.

A gE antigen gene was synthesized, and meanwhile enzymatic cleavage sites were added on both sides of the gene fragment, the specific enzymatic cleavage sites were determined according to the selected recombination system and shuttle plasmid, and this method was mastered by those skilled in the art. The Adeasy vector system was taken as an example to display the construction process of the Ad-SamRNA vaccine hereafter.

Firstly, the gE antigen gene was ligated into the shuttle plasmid pS-C to form pS-C-gE, and the ligation method adopted an experimental method commonly used in the industry. The shuttle plasmid pS-C and the gE antigen gene were subjected to double enzyme digestion with KpnI and XhoI at the same time (see Table 1 below for the enzyme digestion reaction system). After the enzyme digestion reaction was completed, the target fragments were recovered with a gel extraction kit (see the instructions for the recovery method). Then the linearized antigen fragment and the plasmid were ligated by a T4 DNA ligase (see Table 2 below for the reaction system). The construction methods adopting other adenoviral vector systems were similar to the above.

**Table 1: Double-enzyme digestion reaction system (50 µl)**

| Reagent | Volume |
|---|---|
| Enzyme A | 2 µl |
| Enzyme B | 2 µl |
| Buffer | 5 µl |
| Shuttle plasmid or gE gene fragment | 30 µl |
| water | 11 µl |

The double enzyme digestion reaction was carried out at 37°C for at least 4 h or above.

**Table 2 Enzyme ligation reaction system (10 µl)**

| | Reagent | Volume |
|---|---|---|
| | Shuttle plasmid | 2 µl |
| | gE gene fragment | 2 µl |
| | Buffer | 1 µl |
| | T4 DNA ligase | 1 µl |
| H2O | | |

The ligation reaction was carried out at 4°C overnight.

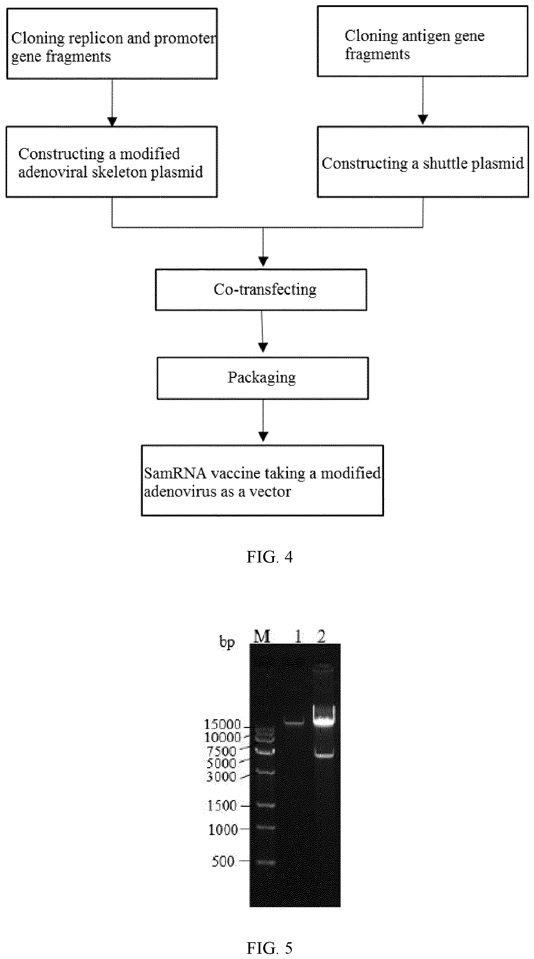

The shuttle plasmid pS-C-gE containing an antigen gene, and the adenoviral skeleton plasmid (such as pAdEasy-1) in which the viral gene replication complex and the promoter were inserted, were used for co-transfection, the two plasmids would undergo homologous recombination, and Ad-SamRNA-gE was obtained through separation. The flow diagram of constructing Ad-SamRNA was shown in FIG. 4.

The constructed Ad-SamRNA-gE was verified by a double enzyme digestion method, and the successfully constructed vector would be used for production and immunogenicity evaluation of the vaccine. The results of double enzyme digestion were as shown in FIG. 5 below: a lane M was a marker, a lane 1 was double enzyme digestion of the adenoviral vector, and a lane 2 was double enzyme digestion of Ad-SamRNA-gE. The results showed that the gE antigen gene was correctly integrated into the adenoviral vector system.

### Embodiment 3 preparation of Ad-SamRNA-gE vaccine

Taking the herpes zoster gE protein antigen as an example, the display of a preparation process of an Ad-SamRNA vaccine was conducted.

An adenoviral vector could massively propagate in 293 cells. HEK293 cells were mostly used for the Admax adenoviral vector system, while AD293 cells were used for Adeasy recombinant adenoviral vector cells.

293 cells were infected with Ad-SamRNA-gE at MOI = 5-10 for at least 40 h, and then centrifuged at 8,000 g for 10 min to collect cell precipitates. The cell precipitates were dissolved in PB or a lysis buffer (2mM MgCl₂, 50mM HEPES, pH 7.5), and then repeatedly frozen and thawed at -80°C for three times for cell lysis, the cell debris was removed by centrifugation, and the supernatant passed through CL-4B, and subjected to one-step chromatography to obtain a target virus.

Besides cell debris, various impure proteins in the cell lysis solution were Ad-SamRNA-gE, and the cell debris could be removed by centrifugation. Compared with impure proteins, the molecular weight of Ad-SamRNA-gE was much larger than those of impure proteins, and therefore, a pure virus could be obtained by a one-step process with CL-4B.

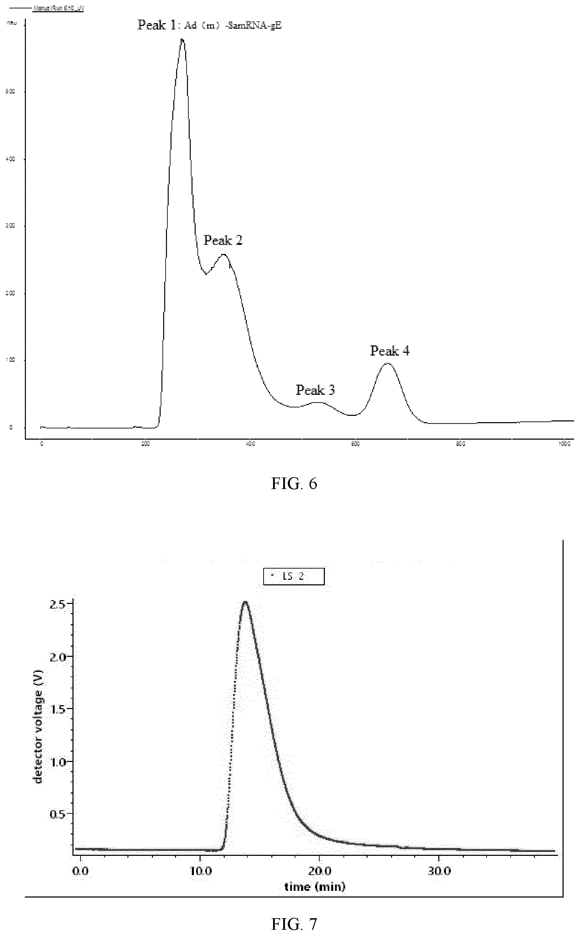

The result of purifying Ad-SamRNA-gE by CL-4B was shown in FIG. 6. The molecular weight of Ad-SamRNA-gE was the largest, and thus was eluted first. The peak 1 in FIG. 6 was Ad-SamRNA-gE.

The purity of the harvested Ad-SamRNA-gE was analyzed by HPLC. A TSK5000 column was selected for the experiment. The HPLC result was shown in FIG. 7. It could be seen that no impurity peak could be seen for the harvested virus, and thus the purity of the virus solution was very high.

### Embodiment 4 study on immunogenicity of Ad-SamRNA-gE vaccine

Taking the herpes zoster gE protein antigen as an example, the display of the immunogenicity of the Ad-SamRNA vaccine was conducted.
An experimental group: an Ad-SamRNA-gE group prepared in Embodiment 3;
A control group 1: a group directly injected with mRNA of the gE protein, which was referred to as mRNA-gE for short;
A control group 2: gE protein antigen gene vaccine using a common adenovirus as a vector, referred to as Ad-gE for short;
A negative control group: a normal saline immunization group
Experimental animals: NIH mice were taken, with 10 in each group, and a weight of 12-14 g.

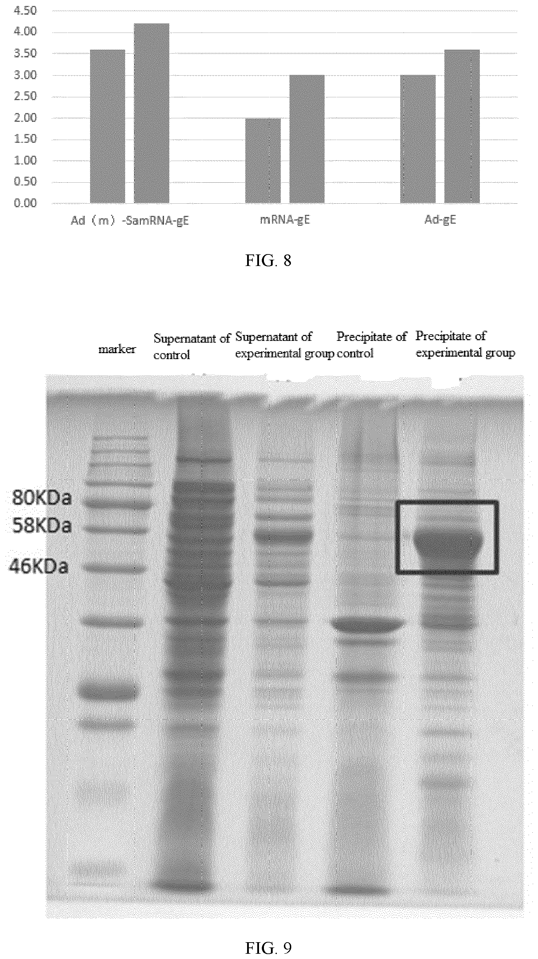

Immunization mode: subcutaneous injection, wherein the experimental group, the control group 1 and the control group 2 were injected with drugs at a single injection dose of 1 × 10⁸ IFU. Each mouse was immunized with two injections, with an interval of 4 weeks between the two injections. Primary immunization blood was collected from the orbit before the immunization with the second injection, and the eyeball was enucleated to collect the secondary immunization serum 28 days after the last immunization. The antibody titer in the serum was determined by ELISA, and the results were shown in FIG. 8.

It could be seen according to the results shown in FIG. 8 that the immunogenicity of Ad-SamRNA-gE was superior to that of Ad-gE and significantly superior to that of mRNA-gE, with significant difference. Therefore, it was proved that the antigen gene vaccine taking the modified adenovirus as the vector, prepared by the present invention, could greatly improve the immunogenicity of the mRNA vaccine.

### Embodiment 5 determination of expression quantities of Ad-SamRNA-gE and Ad-gE

293 cells were infected with the constructed Ad-SamRNA-gE and Ad-gE at MOI = 10, incubated in a 5% CO₂ incubator at 37°C for 40 h, and then centrifuged to collect a cell supernatant and cell precipitates, and the cell precipitates was lysed with a lysis buffer (2mM MgCl₂, 50mM HEPES, pH 7.5); and the expression quantities of the gE protein in the two vectors was analyzed by SDS-PAGE. The results were shown in FIG. 9.

FIG. 9 demonstrated that compared with the control group Ad-gE, the expression quantity of the gE protein was significantly higher after the cells were infected with the sample to be tested Ad-SamRNA-gE. The result corresponded to the case that the immunogenicity of Ad-SamRNA-gE is much higher than that of Ad-gE in Embodiment 4.

### Embodiment 6 immunogenicity of molecular adjuvant against Ad-SamRNA-gE

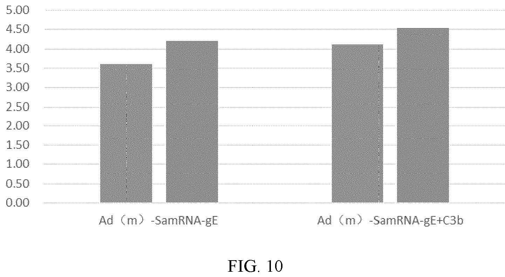

Taking the herpes zoster gE protein antigen as an example, the display of the immunogenicity of the Ad-SamRNA vaccine was conducted.
Experimental groups: Ad-SamRNA-gE with C3b and Ad-SamRNA-gE without C3b were prepared respectively.
A negative control group: a normal saline immunization group
Experimental animals: NIH mice were taken, with 10 in each group, and a weight of 12-14 g.
Immunization mode: subcutaneous injection, wherein the experimental group, the control group 1 and the control group 2 were injected with drugs at a single injection dose of 1 × 10⁸ IFU. Each mouse was immunized with two injections, with an interval of 4 weeks between the two injections. Primary immunization blood was collected from the orbit before the immunization with the second injection, and the eyeball was enucleated to collect the secondary immunization serum 28 days after the last immunization. The antibody titer in the serum was determined by ELISA, and the results were shown in FIG. 10.

According to the results in FIG. 10, C3b could enhance the immunogenicity of Ad-SamRNA-gE.

Finally, it should be noted that the embodiments described above are only illustrative of the technical solutions of the present invention, rather than limiting the present invention; although the present invention is described in detail with reference to the foregoing embodiments, it should be understood by those of ordinary skills in the art that modifications still can be made to the technical solutions described in the foregoing embodiments or equivalent replacements can be made to some or all technical features in the foregoing embodiments; and these modifications and replacements would not make the nature of the corresponding technical solutions depart from the scope of the technical solutions of the embodiments of the present invention.

## Claims

1. An SamRNA vaccine, comprising a recombinant viral vector which comprises: i) a viral gene replication complex comprising nucleotide sequences encoding viral gene replication-related proteins nsP1, nsP2, nsP3, and nsP4; and ii) a nucleotide sequence encoding at least one antigen.

2. The SamRNA vaccine according to claim 1, wherein the recombinant viral vector is a recombinant adenovirus, a chimpanzee adenovirus, a recombinant vesicular stomatitis virus, a recombinant poxvirus, a recombinant dengue virus, a recombinant Kunjin virus, a recombinant sendai virus, or a recombinant canine distemper virus.

3. The SamRNA vaccine according to claim 1, wherein the antigen causes an immune response against bacteria, viruses, fungi or parasites.

4. The SamRNA vaccine according to claim 3, wherein the antigen is a human herpes zoster virus gE protein, a rotavirus VP4 or VP7, an HPV-L1 protein, or an Ebola virus gP protein.

5. The SamRNA vaccine according to claim 1, wherein the antigen is a tumor-specific antigen, and is selected from NY-ESO-1, SSX2, SCP1, RAGE, BAGE, GAGE, MAGE family polypeptides, p53, p21/Ras, CDK4, MUM1, caspase-8, CIA0205, HLA-A2-R1701, β-catenin, TCR, BCR-abl, triosephosphate isomerase, KIA0205, CDC-27, LDLR-FUT, Galectin 4, Galectin 9, protease 3, WT 1, carbonic anhydrase, aldolase A, PRAME, HER-2/neu, mammaglobin, alpha-fetal protein, KSA, gastrin, telomerase catalytic protein, MUC-1, G-250, p53, carcino-embryonic antigens, melanoma-melanocyte differentiation antigens, PAP, PSA, PSMA, PSH-P1, PSM-P1, PSM-P2, p15, Hom/Mel-40, H-Ras, E2A-PRL, H4-RET, IGH-IGK, MYL-RAR, EB viral antigens, EBNA, human papillomavirus antigens, hepatitis B and C viral antigens, human T-lymphotropic viral antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791Tgp72, β-HCG, BCA225, BTAA, CA 125, CA 15-3(CA 27.29\BCAA), CA 195, CA 242, CA-50, CAM43, CD68\KP1, CO-029, FGF-5, Ga733(EpCAM), HTgp-175, M344, MA-50, MG7-Ag, MOV18, NB/70K, NY-CO-1, RCAS1, SDCCAG16, TAAL6, TAG72, TLP, and TPS.

6. The SamRNA vaccine according to claim 1, wherein the recombinant viral vector is obtained by co-transfecting a modified adenoviral skeleton plasmid and a shuttle plasmid containing a nucleotide sequence encoding at least one antigen, wherein the modified adenoviral skeleton plasmid comprises a viral gene replication complex which is gene sequences encoding viral gene replication-related proteins nsP1, nsP2, nsP3 and nsP4.

7. The SamRNA vaccine according to claim 6, wherein the adenoviral skeleton plasmid is selected from pAdEasy-1, pAdEasy-2, pBHG11, pBHG-fiber5 or pBHG-fiber35.

8. The SamRNA vaccine according to claim 1, wherein the recombinant viral vector further comprises: iii) a promoter for transcribing an antigen gene.

9. The SamRNA vaccine according to claim 1, wherein the recombinant viral vector further comprises: iv) a nucleotide sequence encoding at least one adjuvant.

10. The SamRNA vaccine according to claim 8, wherein the adjuvant is selected from C3b, GM-CSF, IL-17, IFN, IL-15, IL-21, IL-1, IL-2, IL-3, IL-4, IL-5, IL-6, IL-7, IL-8, IL-9, IL-10, IL-12, INF-α, INF-γ and CpG.

11. A method for preparing the SamRNA vaccine according to claim 1, comprising the steps of: constructing a modified adenoviral skeleton plasmid; cloning an antigen gene fragment; constructing a shuttle plasmid; co-transfecting and packaging the shuttle plasmid and the adenoviral skeleton plasmid, wherein the modified adenoviral skeleton plasmid comprises a viral gene replication complex which is gene sequences encoding viral gene replication-related proteins nsP1, nsP2, nsP3, and nsP4.

12. A pharmaceutical composition, comprising the SamRNA vaccine according to any one of claims 1-10, and a pharmaceutically-acceptable adjuvant.

13. Use of the SamRNA vaccine according to any one of claims 1-10 or the pharmaceutical composition according to claim 12 in preparation of a vaccine for mammals.

14. A modified adenoviral skeleton plasmid, comprising a viral gene replication complex which is gene sequences encoding viral gene replication-related proteins nsP1, nsP2, nsP3 and nsP4, wherein the adenoviral skeleton plasmid is selected from pAdEasy-1, pAdEasy-2, pBHG11, pBHG-fiber5, or pBHG-fiber35.


### SEQUENCE LISTING

SEQUENCE LISTING

<110>  CANSINO BIOLOGICS INC .

<120>  SamRNA VACCINE AND PREPARATION METHOD THEREFOR

<130>  1

<160>  9

<170>  PatentIn version 3.5

<210>  1
<211>  537
<212>  PRT
<213>  Artificial Sequence

<400>  1

Met Ala Ala Lys Val His Val Asp Ile Glu Ala Asp Ser Pro Phe Ile
1               5                   10                  15

Lys Ser Leu Gln Lys Ala Phe Pro Ser Phe Glu Val Glu Ser Leu Gln
            20                  25                  30

Val Thr Pro Asn Asp His Ala Asn Ala Arg Ala Phe Ser His Leu Ala
        35                  40                  45

Thr Lys Leu Ile Glu Gln Glu Thr Asp Lys Asp Thr Leu Ile Leu Asp
    50                  55                  60

Ile Gly Ser Ala Pro Ser Arg Arg Met Met Ser Thr His Lys Tyr His
65                  70                  75                  80

Cys Val Cys Pro Met Arg Ser Ala Glu Asp Pro Glu Arg Leu Asp Ser
                85                  90                  95

Tyr Ala Lys Lys Leu Ala Ala Ala Ser Gly Lys Val Leu Asp Arg Glu
            100                 105                 110

Ile Ala Gly Lys Ile Thr Asp Leu Gln Thr Val Met Ala Thr Pro Asp
        115                 120                 125
Ala Glu Ser Pro Thr Phe Cys Leu His Thr Asp Val Thr Cys Arg Thr
    130                 135                 140
Ala Ala Glu Val Ala Val Tyr Gln Asp Val Tyr Ala Val His Ala Pro
145                 150                 155                 160
Thr Ser Leu Tyr His Gln Ala Met Lys Gly Val Arg Thr Ala Tyr Trp
                165                 170                 175
Ile Gly Phe Asp Thr Thr Pro Phe Met Phe Asp Ala Leu Ala Gly Ala
            180                 185                 190
Tyr Pro Thr Tyr Ala Thr Asn Trp Ala Asp Glu Gln Val Leu Gln Ala
        195                 200                 205
Arg Asn Ile Gly Leu Cys Ala Ala Ser Leu Thr Glu Gly Arg Leu Gly

    210                 215                 220

Lys Leu Ser Ile Leu Arg Lys Lys Gln Leu Lys Pro Cys Asp Thr Val

225                 230                 235                 240

Met Phe Ser Val Gly Ser Thr Leu Tyr Thr Glu Ser Arg Lys Leu Leu

                245                 250                 255

Arg Ser Trp His Leu Pro Ser Val Phe His Leu Lys Gly Lys Gln Ser

            260                 265                 270

Phe Thr Cys Arg Cys Asp Thr Ile Val Ser Cys Glu Gly Tyr Val Val

        275                 280                 285

Lys Lys Ile Thr Met Cys Pro Gly Leu Tyr Gly Lys Thr Val Gly Tyr

    290                 295                 300

Ala Val Thr Tyr His Ala Glu Gly Phe Leu Val Cys Lys Thr Thr Asp

305                 310                 315                 320

Thr Val Lys Gly Glu Arg Val Ser Phe Pro Val Cys Thr Tyr Val Pro
                325                 330                 335
Ser Thr Ile Cys Asp Gln Met Thr Gly Ile Leu Ala Thr Asp Val Thr
            340                 345                 350
Pro Glu Asp Ala Gln Lys Leu Leu Val Gly Leu Asn Gln Arg Ile Val
        355                 360                 365
Val Asn Gly Arg Thr Gln Arg Asn Thr Asn Thr Met Lys Asn Tyr Leu
    370                 375                 380
Leu Pro Ile Val Ala Val Ala Phe Ser Lys Trp Ala Arg Glu Tyr Lys
385                 390                 395                 400
Ala Asp Leu Asp Asp Glu Lys Pro Leu Gly Val Arg Glu Arg Ser Leu
                405                 410                 415
Thr Cys Cys Cys Leu Trp Ala Phe Lys Thr Arg Lys Met His Thr Met
            420                 425                 430

Tyr Lys Lys Pro Asp Thr Gln Thr Ile Val Lys Val Pro Ser Glu Phe
        435                 440                 445
Asn Ser Phe Val Ile Pro Ser Leu Trp Ser Thr Gly Leu Ala Ile Pro
    450                 455                 460
Val Arg Ser Arg Ile Lys Met Leu Leu Ala Lys Lys Thr Lys Arg Glu
465                 470                 475                 480
Leu Ile Pro Val Leu Asp Ala Ser Ser Ala Arg Asp Ala Glu Gln Glu
                485                 490                 495
Glu Lys Glu Arg Leu Glu Ala Glu Leu Thr Arg Glu Ala Leu Pro Pro
            500                 505                 510
Leu Val Pro Ile Ala Pro Ala Glu Thr Gly Val Val Asp Val Asp Val
        515                 520                 525
Glu Glu Leu Glu Tyr His Ala Gly Ala
    530                 535

<210> 2

<211> 798

<212> PRT

<213> Artificial Sequence

<400> 2

Gly Val Val Glu Thr Pro Arg Ser Ala Leu Lys Val Thr Ala Gln Pro

1               5                   10                  15

Asn Asp Val Leu Leu Gly Asn Tyr Val Val Leu Ser Pro Gln Thr Val

            20                  25                  30

Leu Lys Ser Ser Lys Leu Ala Pro Val His Pro Leu Ala Glu Gln Val

        35                  40                  45

Lys Ile Ile Thr His Asn Gly Arg Ala Gly Gly Tyr Gln Val Asp Gly

    50                  55                  60

Tyr Asp Gly Arg Val Leu Leu Pro Cys Gly Ser Ala Ile Pro Val Pro

65                  70                  75                  80

Glu Phe Gln Ala Leu Ser Glu Ser Ala Thr Met Val Tyr Asn Glu Arg
                85                  90                  95
Glu Phe Val Asn Arg Lys Leu Tyr His Ile Ala Val His Gly Pro Ser
            100                 105                 110
Leu Asn Thr Asp Glu Glu Asn Tyr Glu Lys Val Arg Ala Glu Arg Thr
        115                 120                 125
Asp Ala Glu Tyr Val Phe Asp Val Asp Lys Lys Cys Cys Val Lys Arg
    130                 135                 140
Glu Glu Ala Ser Gly Leu Val Leu Val Gly Glu Leu Thr Asn Pro Pro
145                 150                 155                 160
Phe His Glu Phe Ala Tyr Glu Gly Leu Lys Ile Arg Pro Ser Ala Pro
                165                 170                 175
Tyr Lys Thr Thr Val Val Gly Val Phe Gly Val Pro Gly Ser Gly Lys

            180                 185                 190
Ser Ala Ile Ile Lys Ser Leu Val Thr Lys His Asp Leu Val Thr Ser
        195                 200                 205
Gly Lys Lys Glu Asn Cys Gln Glu Ile Val Asn Asp Val Lys Lys His
    210                 215                 220
Arg Gly Lys Gly Thr Ser Arg Glu Asn Ser Asp Ser Ile Leu Leu Asn
225                 230                 235                 240
Gly Cys Arg Arg Ala Val Asp Ile Leu Tyr Val Asp Glu Ala Phe Ala
                245                 250                 255
Cys His Ser Gly Thr Leu Leu Ala Leu Ile Ala Leu Val Lys Pro Arg
            260                 265                 270
Ser Lys Val Val Leu Cys Gly Asp Pro Lys Gln Cys Gly Phe Phe Asn
        275                 280                 285

Met Met Gln Leu Lys Val Asn Phe Asn His Asn Ile Cys Thr Glu Val
    290                 295                 300
Cys His Lys Ser Ile Ser Arg Arg Cys Thr Arg Pro Val Thr Ala Ile
305                 310                 315                 320
Val Ser Thr Leu His Tyr Gly Gly Lys Met Arg Thr Thr Asn Pro Cys
                325                 330                 335
Asn Lys Pro Ile Ile Ile Asp Thr Thr Gly Gln Thr Lys Pro Lys Pro
            340                 345                 350
Gly Asp Ile Val Leu Thr Cys Phe Arg Gly Trp Ala Lys Gln Leu Gln
        355                 360                 365
Leu Asp Tyr Arg Gly His Glu Val Met Thr Ala Ala Ala Ser Gln Gly
    370                 375                 380
Leu Thr Arg Lys Gly Val Tyr Ala Val Arg Gln Lys Val Asn Glu Asn
385                 390                 395                 400

Pro Leu Tyr Ala Pro Ala Ser Glu His Val Asn Val Leu Leu Thr Arg
                405                 410                 415
Thr Glu Asp Arg Leu Val Trp Lys Thr Leu Ala Gly Asp Pro Trp Ile
            420                 425                 430
Lys Val Leu Ser Asn Ile Pro Gln Gly Asn Phe Thr Ala Thr Leu Glu
        435                 440                 445
Glu Trp Gln Glu Glu His Asp Lys Ile Met Lys Val Ile Glu Gly Pro
    450                 455                 460
Ala Ala Pro Val Asp Ala Phe Gln Asn Lys Ala Asn Val Cys Trp Ala
465                 470                 475                 480
Lys Ser Leu Val Pro Val Leu Asp Thr Ala Gly Ile Arg Leu Thr Ala
                485                 490                 495
Glu Glu Trp Ser Thr Ile Ile Thr Ala Phe Lys Glu Asp Arg Ala Tyr
            500                 505                 510

Ser Pro Val Val Ala Leu Asn Glu Ile Cys Thr Lys Tyr Tyr Gly Val
        515                 520                 525
Asp Leu Asp Ser Gly Leu Phe Ser Ala Pro Lys Val Ser Leu Tyr Tyr
    530                 535                 540
Glu Asn Asn His Trp Asp Asn Arg Pro Gly Gly Arg Met Tyr Gly Phe
545                 550                 555                 560
Asn Ala Ala Thr Ala Ala Arg Leu Glu Ala Arg His Thr Phe Leu Lys
                565                 570                 575
Gly Gln Trp His Thr Gly Lys Gln Ala Val Ile Ala Glu Arg Lys Ile
            580                 585                 590
Gln Pro Leu Ser Val Leu Asp Asn Val Ile Pro Ile Asn Arg Arg Leu
        595                 600                 605
Pro His Ala Leu Val Ala Glu Tyr Lys Thr Val Lys Gly Ser Arg Val

    610                 615                 620

Glu Trp Leu Val Asn Lys Val Arg Gly Tyr His Val Leu Leu Val Ser

625                 630                 635                 640

Glu Tyr Asn Leu Ala Leu Pro Arg Arg Arg Val Thr Trp Leu Ser Pro

                645                 650                 655

Leu Asn Val Thr Gly Ala Asp Arg Cys Tyr Asp Leu Ser Leu Gly Leu

            660                 665                 670

Pro Ala Asp Ala Gly Arg Phe Asp Leu Val Phe Val Asn Ile His Thr

        675                 680                 685

Glu Phe Arg Ile His His Tyr Gln Gln Cys Val Asp His Ala Met Lys

    690                 695                 700

Leu Gln Met Leu Gly Gly Asp Ala Leu Arg Leu Leu Lys Pro Gly Gly

705                 710                 715                 720

Ile Leu Met Arg Ala Tyr Gly Tyr Ala Asp Lys Ile Ser Glu Ala Val
                725                 730                 735
Val Ser Ser Leu Ser Arg Lys Phe Ser Ser Ala Arg Val Leu Arg Pro
            740                 745                 750
Asp Cys Val Thr Ser Asn Thr Glu Val Phe Leu Leu Phe Ser Asn Phe
        755                 760                 765
Asp Asn Gly Lys Arg Pro Ser Thr Leu His Gln Met Asn Thr Lys Leu
    770                 775                 780
Ser Ala Val Tyr Ala Gly Glu Ala Met His Thr Ala Gly Cys
785                 790                 795
<210> 3
<211> 482
<212> PRT
<213> Artificial Sequence
<400> 3

Ala Pro Ser Tyr Arg Val Lys Arg Ala Asp Ile Ala Thr Cys Thr Glu
1               5                   10                  15

Ala Ala Val Val Asn Ala Ala Asn Ala Arg Gly Thr Val Gly Asp Gly
            20                  25                  30

Val Cys Arg Ala Val Ala Lys Lys Trp Pro Ser Ala Phe Lys Gly Ala
        35                  40                  45

Ala Thr Pro Val Gly Thr Ile Lys Thr Val Met Cys Gly Ser Tyr Pro
    50                  55                  60

Val Ile His Ala Val Ala Pro Asn Phe Ser Ala Thr Thr Glu Ala Glu
65                  70                  75                  80

Gly Asp Arg Glu Leu Ala Ala Val Tyr Arg Ala Val Ala Ala Glu Val
                85                  90                  95

Asn Arg Leu Ser Leu Ser Ser Val Ala Ile Pro Leu Leu Ser Thr Gly
            100                 105                 110

Val Phe Ser Gly Gly Arg Asp Arg Leu Gln Gln Ser Leu Asn His Leu
        115                 120                 125
Phe Thr Ala Met Asp Ala Thr Asp Ala Asp Val Thr Ile Tyr Cys Arg
    130                 135                 140
Asp Lys Ser Trp Glu Lys Lys Ile Gln Glu Ala Ile Asp Met Arg Thr
145                 150                 155                 160
Ala Val Glu Leu Leu Asn Asp Asp Val Glu Leu Thr Thr Asp Leu Val
                165                 170                 175
Arg Val His Pro Asp Ser Ser Leu Val Gly Arg Lys Gly Tyr Ser Thr
            180                 185                 190
Thr Asp Gly Ser Leu Tyr Ser Tyr Phe Glu Gly Thr Lys Phe Asn Gln
        195                 200                 205
Ala Ala Ile Asp Met Ala Glu Ile Leu Thr Leu Trp Pro Arg Leu Gln
    210                 215                 220

Glu Ala Asn Glu Arg Ile Cys Leu Tyr Ala Leu Gly Glu Thr Met Asp
225                 230                 235                 240
Asn Ile Gly Ser Lys Cys Pro Val Asn Asp Ser Asp Ser Ser Thr Pro
                245                 250                 255
Pro Arg Thr Val Pro Cys Leu Cys Arg Tyr Ala Met Thr Ala Glu Arg
            260                 265                 270
Ile Ala Arg Leu Arg Ser His Gln Val Lys Ser Met Val Val Cys Ser
        275                 280                 285
Ser Phe Pro Leu Pro Lys Tyr His Val Asp Gly Val Gln Lys Val Lys
    290                 295                 300
Cys Glu Lys Val Leu Leu Phe Asp Pro Thr Val Pro Ser Val Val Ser
305                 310                 315                 320
Pro Arg Lys Tyr Ala Ala Ser Thr Thr Asp His Ser Asp Arg Ser Leu

                325                 330                 335

Arg Gly Phe Asp Leu Asp Trp Thr Thr Asp Ser Ser Ser Thr Ala Ser

            340                 345                 350

Asp Thr Met Ser Leu Pro Ser Leu Gln Ser Cys Asp Ile Asp Ser Ile

        355                 360                 365

Tyr Glu Pro Met Ala Pro Ile Val Val Thr Ala Asp Val His Pro Glu

    370                 375                 380

Pro Ala Gly Ile Ala Asp Leu Ala Ala Asp Val His Pro Glu Pro Ala

385                 390                 395                 400

Asp His Val Asp Leu Glu Asn Pro Ile Pro Pro Pro Arg Pro Lys Arg

                405                 410                 415

Ala Ala Tyr Leu Ala Ser Arg Ala Ala Glu Arg Pro Val Pro Ala Pro

            420                 425                 430

Arg Lys Pro Thr Pro Ala Pro Arg Thr Ala Phe Arg Asn Lys Leu Pro
        435                 440                 445
Leu Thr Phe Gly Asp Phe Asp Glu His Glu Val Asp Ala Leu Ala Ser
    450                 455                 460
Gly Ile Thr Phe Gly Asp Phe Asp Asp Val Leu Arg Leu Gly Arg Ala
465                 470                 475                 480
Gly Ala
<210> 4
<211> 614
<212> PRT
<213> Artificial Sequence
<400> 4
Tyr Ile Phe Ser Ser Asp Thr Gly Ser Gly His Leu Gln Gln Lys Ser
1               5                   10                  15

Val Arg Gln His Asn Leu Gln Cys Ala Gln Leu Asp Ala Val Gln Glu
            20                  25                  30
Glu Lys Met Tyr Pro Pro Lys Leu Asp Thr Glu Arg Glu Lys Leu Leu
        35                  40                  45
Leu Leu Lys Met Gln Met His Pro Ser Glu Ala Asn Lys Ser Arg Tyr
    50                  55                  60
Gln Ser Arg Lys Val Glu Asn Met Lys Ala Thr Val Val Asp Arg Leu
65                  70                  75                  80
Thr Ser Gly Ala Arg Leu Tyr Thr Gly Ala Asp Val Gly Arg Ile Pro
                85                  90                  95
Thr Tyr Ala Val Arg Tyr Pro Arg Pro Val Tyr Ser Pro Thr Val Ile
            100                 105                 110
Glu Arg Phe Ser Ser Pro Asp Val Ala Ile Ala Ala Cys Asn Glu Tyr
        115                 120                 125

Leu Ser Arg Asn Tyr Pro Thr Val Ala Ser Tyr Gln Ile Thr Asp Glu
    130                 135                 140
Tyr Asp Ala Tyr Leu Asp Met Val Asp Gly Ser Asp Ser Cys Leu Asp
145                 150                 155                 160
Arg Ala Thr Phe Cys Pro Ala Lys Leu Arg Cys Tyr Pro Lys His His
                165                 170                 175
Ala Tyr His Gln Pro Thr Val Arg Ser Ala Val Pro Ser Pro Phe Gln
            180                 185                 190
Asn Thr Leu Gln Asn Val Leu Ala Ala Ala Thr Lys Arg Asn Cys Asn
        195                 200                 205
Val Thr Gln Met Arg Glu Leu Pro Thr Met Asp Ser Ala Val Phe Asn
    210                 215                 220
Val Glu Cys Phe Lys Arg Tyr Ala Cys Ser Gly Glu Tyr Trp Glu Glu
225                 230                 235                 240

Tyr Ala Lys Gln Pro Ile Arg Ile Thr Thr Glu Asn Ile Thr Thr Tyr
                245                 250                 255
Val Thr Lys Leu Lys Gly Pro Lys Ala Ala Ala Leu Phe Ala Lys Thr
            260                 265                 270
His Asn Leu Val Pro Leu Gln Glu Val Pro Met Asp Arg Phe Thr Val
        275                 280                 285
Asp Met Lys Arg Asp Val Lys Val Thr Pro Gly Thr Lys His Thr Glu
    290                 295                 300
Glu Arg Pro Lys Val Gln Val Ile Gln Ala Ala Glu Pro Leu Ala Thr
305                 310                 315                 320
Ala Tyr Leu Cys Gly Ile His Arg Glu Leu Val Arg Arg Leu Asn Ala
                325                 330                 335
Val Leu Arg Pro Asn Val His Thr Leu Phe Asp Met Ser Ala Glu Asp

            340                 345                 350
Phe Asp Ala Ile Ile Ala Ser His Phe His Pro Gly Asp Pro Val Leu
        355                 360                 365
Glu Thr Asp Ile Ala Ser Phe Asp Lys Ser Gln Asp Asp Ser Leu Ala
    370                 375                 380
Leu Thr Gly Leu Met Ile Leu Glu Asp Leu Gly Val Asp Gln Tyr Leu
385                 390                 395                 400
Leu Asp Leu Ile Glu Ala Ala Phe Gly Glu Ile Ser Ser Cys His Leu
                405                 410                 415
Pro Thr Gly Thr Arg Phe Lys Phe Gly Ala Met Met Lys Ser Gly Met
            420                 425                 430
Phe Leu Thr Leu Phe Ile Asn Thr Val Leu Asn Ile Thr Ile Ala Ser
        435                 440                 445

Arg Val Leu Glu Gln Arg Leu Thr Asp Ser Ala Cys Ala Ala Phe Ile
    450                 455                 460
Gly Asp Asp Asn Ile Val His Gly Val Ile Ser Asp Lys Leu Met Ala
465                 470                 475                 480
Glu Arg Cys Ala Ser Trp Val Asn Met Glu Val Lys Ile Ile Asp Ala
                485                 490                 495
Val Met Gly Glu Lys Pro Pro Tyr Phe Cys Gly Gly Phe Ile Val Phe
            500                 505                 510
Asp Ser Val Thr Gln Thr Ala Cys Arg Val Ser Asp Pro Leu Lys Arg
        515                 520                 525
Leu Phe Lys Leu Gly Lys Pro Leu Thr Ala Glu Asp Lys Gln Asp Glu
    530                 535                 540
Asp Arg Arg Arg Ala Leu Ser Asp Glu Val Ser Lys Trp Phe Arg Thr
545                 550                 555                 560

Gly Leu Gly Ala Glu Leu Glu Val Ala Leu Thr Ser Arg Tyr Glu Val
                565                 570                 575

Glu Gly Cys Lys Ser Ile Leu Ile Ala Met Thr Thr Leu Ala Arg Asp
            580                 585                 590

Ile Lys Ala Phe Lys Lys Leu Arg Gly Pro Val Ile His Leu Tyr Gly
        595                 600                 605

Gly Pro Arg Leu Val Arg
    610

<210> 5
<211> 193
<212> DNA
<213> Artificial Sequence

<400> 5
gagtggacct gtgtcgcgta aatctaaaag taagagtgag gcagaatctt tttcggatag 60

tggcgcttct gagccactaa gttcataatc aagatgtctt actctacttc tggtttgcgt 120

tctttgcctg catatactaa gtctttttgt ccttattatg ctttgtatga tctgttggtg 180

tcagcccaag gtg
193

<210> 6
<211> 1614
<212> DNA
<213> Artificial Sequence

<400> 6
atggccgcca aagtgcatgt tgatattgag gctgacagcc cattcatcaa gtctttgcag 60

aaggcatttc cgtcgttcga ggtggagtca ttgcaggtca caccaaatga ccatgcaaat 120

gccagagcat tttcgcacct ggctaccaaa ttgatcgagc aggagactga caaagacaca 180

ctcatcttgg atatcggcag tgcgccttcc aggagaatga tgtctacgca caaataccac 240

tgcgtatgcc ctatgcgcag cgcagaagac cccgaaaggc tcgatagcta cgcaaagaaa 300

ctggcagcgg cctccgggaa ggtgctggat agagagatcg caggaaaaat caccgacctg 360

cagaccgtca tggctacgcc agacgctgaa tctcctacct tttgcctgca tacagacgtc 420

acgtgtcgta cggcagccga agtggccgta taccaggacg tgtatgctgt acatgcacca 480

acatcgctgt accatcaggc gatgaaaggt gtcagaacgg cgtattggat tgggtttgac 540

accaccccgt ttatgtttga cgcgctagca ggcgcgtatc caacctacgc cacaaactgg 600

gccgacgagc aggtgttaca ggccaggaac ataggactgt gtgcagcatc cttgactgag 660

ggaagactcg gcaaactgtc cattctccgc aagaagcaat tgaaaccttg cgacacagtc 720

atgttctcgg taggatctac attgtacact gagagcagaa agctactgag gagctggcac 780

ttaccctccg tattccacct gaaaggtaaa caatccttta cctgtaggtg cgataccatc 840

gtatcatgtg aagggtacgt agttaagaaa atcactatgt gccccggcct gtacggtaaa 900

acggtagggt acgccgtgac gtatcacgcg gagggattcc tagtgtgcaa gaccacagac 960

actgtcaaag gagaaagagt ctcattccct gtatgcacct acgtcccctc aaccatctgt 1020

gatcaaatga ctggcatact agcgaccgac gtcacaccgg aggacgcaca gaagttgtta 1080

gtgggattga atcagaggat agttgtgaac ggaagaacac agcgaaacac taacacgatg 1140

aagaactatc tgcttccgat tgtggccgtc gcatttagca agtgggcgag ggaatacaag 1200

gcagaccttg atgatgaaaa acctctgggt gtccgagaga ggtcacttac ttgctgctgc 1260

ttgtgggcat ttaaaacgag gaagatgcac accatgtaca agaaaccaga cacccagaca 1320

atagtgaagg tgccttcaga gtttaactcg ttcgtcatcc cgagcctatg gtctacaggc 1380

ctcgcaatcc cagtcagatc acgcattaag atgcttttgg ccaagaagac caagcgagag 1440

ttaatacctg ttctcgacgc gtcgtcagcc agggatgctg aacaagagga gaaggagagg 1500

ttggaggccg agctgactag agaagcctta ccacccctcg tccccatcgc gccggcggag 1560

acgggagtcg tcgacgtcga cgttgaagaa ctagagtatc acgcaggtgc ataa 1614

<210> 7

<211> 2400

<212> DNA

<213> Artificial Sequence

<400> 7

atgggggtcg tggaaacacc tcgcagcgcg ttgaaagtca ccgcacagcc gaacgacgta 60

ctactaggaa attacgtagt tctgtccccg cagaccgtgc tcaagagctc caagttggcc 120

cccgtgcacc ctctagcaga gcaggtgaaa ataataacac ataacgggag ggccggcggt 180

taccaggtcg acggatatga cggcagggtc ctactaccat gtggatcggc cattccggtc 240

cctgagtttc aagctttgag cgagagcgcc actatggtgt acaacgaaag ggagttcgtc 300

aacaggaaac tataccatat tgccgttcac ggaccgtcgc tgaacaccga cgaggagaac 360

tacgagaaag tcagagctga aagaactgac gccgagtacg tgttcgacgt agataaaaaa 420

tgctgcgtca agagagagga agcgtcgggt ttggtgttgg tgggagagct aaccaacccc 480

ccgttccatg aattcgccta cgaagggctg aagatcaggc cgtcggcacc atataagact 540

acagtagtag gagtctttgg ggttccggga tcaggcaagt ctgctattat taagagcctc 600

gtgaccaaac acgatctggt caccagcggc aagaaggaga actgccagga aatagttaac 660

gacgtgaaga agcaccgcgg gaaggggaca agtagggaaa acagtgactc catcctgcta 720

aacgggtgtc gtcgtgccgt ggacatccta tatgtggacg aggctttcgc ttgccattcc 780

ggtactctgc tggccctaat tgctcttgtt aaacctcgga gcaaagtggt gttatgcgga 840

gaccccaagc aatgcggatt cttcaatatg atgcagctta aggtgaactt caaccacaac 900

atctgcactg aagtatgtca taaaagtata tccagacgtt gcacgcgtcc agtcacggcc 960

atcgtgtcta cgttgcacta cggaggcaag atgcgcacga ccaacccgtg caacaaaccc 1020

ataatcatag acaccacagg acagaccaag cccaagccag gagacatcgt gttaacatgc 1080

ttccgaggct gggcaaagca gctgcagttg gactaccgtg gacacgaagt catgacagca 1140

gcagcatctc agggcctcac ccgcaaaggg gtatacgccg taaggcagaa ggtgaatgaa 1200

aatcccttgt atgcccctgc gtcggagcac gtgaatgtac tgctgacgcg cactgaggat 1260

aggctggtgt ggaaaacgct ggccggcgat ccctggatta aggtcctatc aaacattcca 1320

cagggtaact ttacggccac attggaagaa tggcaagaag aacacgacaa aataatgaag 1380

gtgattgaag gaccggctgc gcctgtggac gcgttccaga acaaagcgaa cgtgtgttgg 1440

gcgaaaagcc tggtgcctgt cctggacact gccggaatca gattgacagc agaggagtgg 1500

agcaccataa ttacagcatt taaggaggac agagcttact ctccagtggt ggccttgaat 1560

gaaatttgca ccaagtacta tggagttgac ctggacagtg gcctgttttc tgccccgaag 1620

gtgtccctgt attacgagaa caaccactgg gataacagac ctggtggaag gatgtatgga 1680

ttcaatgccg caacagctgc caggctggaa gctagacata ccttcctgaa ggggcagtgg 1740

catacgggca agcaggcagt tatcgcagaa agaaaaatcc aaccgctttc tgtgctggac 1800

aatgtaattc ctatcaaccg caggctgccg cacgccctgg tggctgagta caagacggtt 1860

aaaggcagta gggttgagtg gctggtcaat aaagtaagag ggtaccacgt cctgctggtg 1920

agtgagtaca acctggcttt gcctcgacgc agggtcactt ggttgtcacc gctgaatgtc 1980

acaggcgccg ataggtgcta cgacctaagt ttaggactgc cggctgacgc cggcaggttc 2040

gacttggtct ttgtgaacat tcacacggaa ttcagaatcc accactacca gcagtgtgtc 2100

gaccacgcca tgaagctgca gatgcttggg ggagatgcgc tacgactgct aaaacccggc 2160

ggcatcttga tgagagctta cggatacgcc gataaaatca gcgaagccgt tgtttcctcc 2220

ttaagcagaa agttctcgtc tgcaagagtg ttgcgcccgg attgtgtcac cagcaataca 2280

gaagtgttct tgctgttctc caactttgac aacggaaaga gaccctctac gctacaccag 2340

atgaatacca agctgagtgc cgtgtatgcc ggagaagcca tgcacacggc cgggtgttaa 2400

<210> 8

<211> 1452

<212> DNA

<213> Artificial Sequence

<400> 8

atggcaccat cctacagagt taagagagca gacatagcca cgtgcacaga agcggctgtg 60

gttaacgcag ctaacgcccg tggaactgta ggggatggcg tatgcagggc cgtggcgaag 120

aaatggccgt cagcctttaa gggagcagca acaccagtgg gcacaattaa aacagtcatg 180

tgcggctcgt accccgtcat ccacgctgta gcgcctaatt tctctgccac gactgaagcg 240

gaaggggacc gcgaattggc cgctgtctac cgggcagtgg ccgccgaagt aaacagactg 300

tcactgagca gcgtagccat cccgctgctg tccacaggag tgttcagcgg cggaagagat 360

aggctgcagc aatccctcaa ccatctattc acagcaatgg acgccacgga cgctgacgtg 420

accatctact gcagagacaa aagttgggag aagaaaatcc aggaagccat tgacatgagg 480

acggctgtgg agttgctcaa tgatgacgtg gagctgacca cagacttggt gagagtgcac 540

ccggacagca gcctggtggg tcgtaagggc tacagtacca ctgacgggtc gctgtactcg 600

tactttgaag gtacgaaatt caaccaggct gctattgata tggcagagat actgacgttg 660

tggcccagac tgcaggaggc aaacgaacgg atatgcctat acgcgctggg cgaaacaatg 720

gacaacatcg gatccaaatg tccggtgaac gattccgatt catcaacacc tcccaggaca 780

gtgccctgcc tgtgccgcta cgcaatgaca gcagaacgga tcgcccgcct taggtcacac 840

caagttaaaa gcatggtggt ttgctcatct tttcccctcc cgaaatacca tgtagatggg 900

gtgcagaagg taaagtgcga gaaggttctc ctgttcgacc cgacggtacc ttcagtggtt 960

agtccgcgga agtatgccgc atctacgacg gaccactcag atcggtcgtt acgagggttt 1020

gacttggact ggaccaccga ctcgtcttcc actgccagcg ataccatgtc gctacccagt 1080

ttgcagtcgt gtgacatcga ctcgatctac gagccaatgg ctcccatagt agtgacggct 1140

gacgtacacc ctgaacccgc aggcatcgcg gacctggcgg cagatgtgca ccctgaaccc 1200

gcagaccatg tggacctcga gaacccgatt cctccaccgc gcccgaagag agctgcatac 1260

cttgcctccc gcgcggcgga gcgaccggtg ccggcgccga gaaagccgac gcctgcccca
1320

aggactgcgt ttaggaacaa gctgcctttg acgttcggcg actttgacga gcacgaggtc 1380

gatgcgttgg cctccgggat tactttcgga gacttcgacg acgtcctgcg actaggccgc 1440

gcgggtgcat aa
1452

<210> 9
<211> 1908
<212> DNA

<213>  Artificial Sequence

<400>  9

atgtatattt tctcctcgga cactggcagc ggacatttac aacaaaaatc cgttaggcag    60

cacaatctcc agtgcgcaca actggatgcg gtccaggagg agaaaatgta cccgccaaaa    120

ttggatactg agagggagaa gctgttgctg ctgaaaatgc agatgcaccc atcggaggct    180

aataagagtc gataccagtc tcgcaaagtg gagaacatga aagccacggt ggtggacagg    240

ctcacatcgg gggccagatt gtacacggga gcggacgtag gccgcatacc aacatacgcg    300

gttcggtacc cccgccccgt gtactcccct accgtgatcg aaagattctc aagccccgat    360

gtagcaatcg cagcgtgcaa cgaataccta tccagaaatt acccaacagt ggcgtcgtac    420

cagataacag atgaatacga cgcatacttg gacatggttg acgggtcgga tagttgcttg    480

gacagagcga cattctgccc ggcgaagctc cggtgctacc cgaaacatca tgcgtaccac    540

cagccgactg tacgcagtgc cgtcccgtca ccctttcaga acacactaca gaacgtgcta    600

gcggccgcca ccaagagaaa ctgcaacgtc acgcaaatgc gagaactacc caccatggac    660

tcggcagtgt tcaacgtgga gtgcttcaag cgctatgcct gctccggaga atattgggaa    720

gaatatgcta aacaacctat ccggataacc actgagaaca tcactaccta tgtgaccaaa 780

ttgaaaggcc cgaaagctgc tgccttgttc gctaagaccc acaacttggt tccgctgcag 840

gaggttccca tggacagatt cacggtcgac atgaaacgag atgtcaaagt cactccaggg 900

acgaaacaca cagaggaaag acccaaagtc caggtaattc aagcagcgga gccattggcg 960

accgcttacc tgtgcggcat ccacagggaa ttagtaagga gactaaatgc tgtgttacgc 1020

cctaacgtgc acacattgtt tgatatgtcg gccgaagact ttgacgcgat catcgcctct 1080

cacttccacc caggagaccc ggttctagag acggacattg catcattcga caaaagccag 1140

gacgactcct tggctcttac aggtttaatg atcctcgaag atctaggggt ggatcagtac 1200

ctgctggact tgatcgaggc agcctttggg gaaatatcca gctgtcacct accaactggc 1260

acgcgcttca agttcggagc tatgatgaaa tcgggcatgt ttctgacttt gtttattaac 1320

actgttttga acatcaccat agcaagcagg gtactggagc agagactcac tgactccgcc 1380

tgtgcggcct tcatcggcga cgacaacatc gttcacggag tgatctccga caagctgatg 1440

gcggagaggt gcgcgtcgtg ggtcaacatg gaggtgaaga tcattgacgc tgtcatgggc 1500

gaaaaacccc catatttttg tgggggattc atagtttttg acagcgtcac acagaccgcc 1560

tgccgtgttt cagacccact taagcgcctg ttcaagttgg gtaagccgct aacagctgaa 1620

gacaagcagg acgaagacag gcgacgagca ctgagtgacg aggttagcaa gtggttccgg 1680

acaggcttgg gggccgaact ggaggtggca ctaacatcta ggtatgaggt agagggctgc 1740

aaaagtatcc tcatagccat gaccaccttg gcgagggaca ttaaggcgtt taagaaattg 1800

agaggacctg ttatacacct ctacggcggt cctagattgg tgcgttaata cacagaattc 1860

tgattatagc gcactattat agcaccatga attacatccc tacgctaa 1908

SEQUENCE LISTING

<110>  CANSINO BIOLOGICS INC .

<120>   SamRNA VACCINE AND PREPARATION METHOD THEREFOR

<130>  1

<160>  9

<170>  PatentIn version 3.5

<210>  1
<211>  537
<212>  PRT
<213>  Artificial Sequence

<400>  1

Met Ala Ala Lys Val His Val Asp Ile Glu Ala Asp Ser Pro Phe Ile
1               5                   10                  15

Lys Ser Leu Gln Lys Ala Phe Pro Ser Phe Glu Val Glu Ser Leu Gln
            20                  25                  30

Val Thr Pro Asn Asp His Ala Asn Ala Arg Ala Phe Ser His Leu Ala
        35                  40                  45

Thr Lys Leu Ile Glu Gln Glu Thr Asp Lys Asp Thr Leu Ile Leu Asp
    50                  55                  60

Ile Gly Ser Ala Pro Ser Arg Arg Met Met Ser Thr His Lys Tyr His
65                  70                  75                  80

Cys Val Cys Pro Met Arg Ser Ala Glu Asp Pro Glu Arg Leu Asp Ser
                85                  90                  95

Tyr Ala Lys Lys Leu Ala Ala Ala Ser Gly Lys Val Leu Asp Arg Glu
            100                 105                 110

Ile Ala Gly Lys Ile Thr Asp Leu Gln Thr Val Met Ala Thr Pro Asp
        115                 120                 125

Ala Glu Ser Pro Thr Phe Cys Leu His Thr Asp Val Thr Cys Arg Thr
    130                 135                 140

Ala Ala Glu Val Ala Val Tyr Gln Asp Val Tyr Ala Val His Ala Pro
145                 150                 155                 160

Thr Ser Leu Tyr His Gln Ala Met Lys Gly Val Arg Thr Ala Tyr Trp
                165                 170                 175

Ile Gly Phe Asp Thr Thr Pro Phe Met Phe Asp Ala Leu Ala Gly Ala
        180                 185                 190

Tyr Pro Thr Tyr Ala Thr Asn Trp Ala Asp Glu Gln Val Leu Gln Ala
    195                 200                 205

Arg Asn Ile Gly Leu Cys Ala Ala Ser Leu Thr Glu Gly Arg Leu Gly
    210                 215                 220

Lys Leu Ser Ile Leu Arg Lys Lys Gln Leu Lys Pro Cys Asp Thr Val
225                 230                 235                 240

Met Phe Ser Val Gly Ser Thr Leu Tyr Thr Glu Ser Arg Lys Leu Leu
                245                 250                 255

Arg Ser Trp His Leu Pro Ser Val Phe His Leu Lys Gly Lys Gln Ser
        260                 265                 270

Phe Thr Cys Arg Cys Asp Thr Ile Val Ser Cys Glu Gly Tyr Val Val
    275                 280                 285

Lys Lys Ile Thr Met Cys Pro Gly Leu Tyr Gly Lys Thr Val Gly Tyr
    290                 295                 300

Ala Val Thr Tyr His Ala Glu Gly Phe Leu Val Cys Lys Thr Thr Asp
305                 310                 315                 320

Thr Val Lys Gly Glu Arg Val Ser Phe Pro Val Cys Thr Tyr Val Pro
                325                 330                 335

Ser Thr Ile Cys Asp Gln Met Thr Gly Ile Leu Ala Thr Asp Val Thr
        340                 345                 350

Pro Glu Asp Ala Gln Lys Leu Leu Val Gly Leu Asn Gln Arg Ile Val
    355                 360                 365

Val Asn Gly Arg Thr Gln Arg Asn Thr Asn Thr Met Lys Asn Tyr Leu
    370                 375                 380

Leu Pro Ile Val Ala Val Ala Phe Ser Lys Trp Ala Arg Glu Tyr Lys
385                 390                 395                 400

Ala Asp Leu Asp Asp Glu Lys Pro Leu Gly Val Arg Glu Arg Ser Leu
                405                 410                 415

Thr Cys Cys Cys Leu Trp Ala Phe Lys Thr Arg Lys Met His Thr Met
        420                 425                 430

Tyr Lys Lys Pro Asp Thr Gln Thr Ile Val Lys Val Pro Ser Glu Phe
        435                 440                 445

Asn Ser Phe Val Ile Pro Ser Leu Trp Ser Thr Gly Leu Ala Ile Pro
    450                 455                 460

Val Arg Ser Arg Ile Lys Met Leu Leu Ala Lys Lys Thr Lys Arg Glu
465                 470                 475                 480

Leu Ile Pro Val Leu Asp Ala Ser Ser Ala Arg Asp Ala Glu Gln Glu
                485                 490                 495

Glu Lys Glu Arg Leu Glu Ala Glu Leu Thr Arg Glu Ala Leu Pro Pro
            500                 505                 510

Leu Val Pro Ile Ala Pro Ala Glu Thr Gly Val Val Asp Val Asp Val
        515                 520                 525

Glu Glu Leu Glu Tyr His Ala Gly Ala
    530                 535

<210>  2
<211>  798
<212>  PRT
<213>  Artificial Sequence

<400>  2

Gly Val Val Glu Thr Pro Arg Ser Ala Leu Lys Val Thr Ala Gln Pro
1               5                   10                  15

Asn Asp Val Leu Leu Gly Asn Tyr Val Val Leu Ser Pro Gln Thr Val
            20                  25                  30

Leu Lys Ser Ser Lys Leu Ala Pro Val His Pro Leu Ala Glu Gln Val
        35                  40                  45

Lys Ile Ile Thr His Asn Gly Arg Ala Gly Gly Tyr Gln Val Asp Gly
    50                  55                  60

Tyr Asp Gly Arg Val Leu Leu Pro Cys Gly Ser Ala Ile Pro Val Pro
65                  70                  75                  80

Glu Phe Gln Ala Leu Ser Glu Ser Ala Thr Met Val Tyr Asn Glu Arg
                85                  90                  95

Glu Phe Val Asn Arg Lys Leu Tyr His Ile Ala Val His Gly Pro Ser
            100                 105                 110

Leu Asn Thr Asp Glu Glu Asn Tyr Glu Lys Val Arg Ala Glu Arg Thr
        115                 120                 125

Asp Ala Glu Tyr Val Phe Asp Val Asp Lys Lys Cys Cys Val Lys Arg
    130                 135                 140

Glu Glu Ala Ser Gly Leu Val Leu Val Gly Glu Leu Thr Asn Pro Pro
145                 150                 155                 160

Phe His Glu Phe Ala Tyr Glu Gly Leu Lys Ile Arg Pro Ser Ala Pro
                165                 170                 175

Tyr Lys Thr Thr Val Val Gly Val Phe Gly Val Pro Gly Ser Gly Lys
            180                 185                 190

Ser Ala Ile Ile Lys Ser Leu Val Thr Lys His Asp Leu Val Thr Ser
        195                 200                 205

Gly Lys Lys Glu Asn Cys Gln Glu Ile Val Asn Asp Val Lys Lys His
    210                 215                 220

Arg Gly Lys Gly Thr Ser Arg Glu Asn Ser Asp Ser Ile Leu Leu Asn
225                 230                 235                 240

Gly Cys Arg Arg Ala Val Asp Ile Leu Tyr Val Asp Glu Ala Phe Ala
                245                 250                 255

Cys His Ser Gly Thr Leu Leu Ala Leu Ile Ala Leu Val Lys Pro Arg
            260                 265                 270

Ser Lys Val Val Leu Cys Gly Asp Pro Lys Gln Cys Gly Phe Phe Asn
        275                 280                 285

Met Met Gln Leu Lys Val Asn Phe Asn His Asn Ile Cys Thr Glu Val
    290                 295                 300

Cys His Lys Ser Ile Ser Arg Arg Cys Thr Arg Pro Val Thr Ala Ile
305                 310                 315                 320

Val Ser Thr Leu His Tyr Gly Gly Lys Met Arg Thr Thr Asn Pro Cys
                325                 330                 335

Asn Lys Pro Ile Ile Ile Asp Thr Thr Gly Gln Thr Lys Pro Lys Pro
            340                 345                 350

Gly Asp Ile Val Leu Thr Cys Phe Arg Gly Trp Ala Lys Gln Leu Gln
        355                 360                 365

Leu Asp Tyr Arg Gly His Glu Val Met Thr Ala Ala Ala Ser Gln Gly
    370                 375                 380

Leu Thr Arg Lys Gly Val Tyr Ala Val Arg Gln Lys Val Asn Glu Asn
385                 390                 395                 400

Pro Leu Tyr Ala Pro Ala Ser Glu His Val Asn Val Leu Leu Thr Arg
                405                 410                 415

Thr Glu Asp Arg Leu Val Trp Lys Thr Leu Ala Gly Asp Pro Trp Ile
            420                 425                 430

Lys Val Leu Ser Asn Ile Pro Gln Gly Asn Phe Thr Ala Thr Leu Glu
        435                 440                 445

Glu Trp Gln Glu Glu His Asp Lys Ile Met Lys Val Ile Glu Gly Pro
    450                 455                 460

Ala Ala Pro Val Asp Ala Phe Gln Asn Lys Ala Asn Val Cys Trp Ala
465                 470                 475                 480

Lys Ser Leu Val Pro Val Leu Asp Thr Ala Gly Ile Arg Leu Thr Ala
                485                 490                 495

Glu Glu Trp Ser Thr Ile Ile Thr Ala Phe Lys Glu Asp Arg Ala Tyr
            500                 505                 510

Ser Pro Val Val Ala Leu Asn Glu Ile Cys Thr Lys Tyr Tyr Gly Val
        515                 520                 525

Asp Leu Asp Ser Gly Leu Phe Ser Ala Pro Lys Val Ser Leu Tyr Tyr
    530                 535                 540

Glu Asn Asn His Trp Asp Asn Arg Pro Gly Gly Arg Met Tyr Gly Phe
545                 550                 555                 560

Asn Ala Ala Thr Ala Ala Arg Leu Glu Ala Arg His Thr Phe Leu Lys
                565                 570                 575

Gly Gln Trp His Thr Gly Lys Gln Ala Val Ile Ala Glu Arg Lys Ile
            580                 585                 590

Gln Pro Leu Ser Val Leu Asp Asn Val Ile Pro Ile Asn Arg Arg Leu
        595                 600                 605

Pro His Ala Leu Val Ala Glu Tyr Lys Thr Val Lys Gly Ser Arg Val

610                 615                 620

Glu Trp Leu Val Asn Lys Val Arg Gly Tyr His Val Leu Leu Val Ser
625                 630                 635                 640

Glu Tyr Asn Leu Ala Leu Pro Arg Arg Arg Val Thr Trp Leu Ser Pro
                645                 650                 655

Leu Asn Val Thr Gly Ala Asp Arg Cys Tyr Asp Leu Ser Leu Gly Leu
            660                 665                 670

Pro Ala Asp Ala Gly Arg Phe Asp Leu Val Phe Val Asn Ile His Thr
        675                 680                 685

Glu Phe Arg Ile His His Tyr Gln Gln Cys Val Asp His Ala Met Lys
    690                 695                 700

Leu Gln Met Leu Gly Gly Asp Ala Leu Arg Leu Leu Lys Pro Gly Gly
705                 710                 715                 720

Ile Leu Met Arg Ala Tyr Gly Tyr Ala Asp Lys Ile Ser Glu Ala Val
                725                 730                 735

Val Ser Ser Leu Ser Arg Lys Phe Ser Ser Ala Arg Val Leu Arg Pro
            740                 745                 750

Asp Cys Val Thr Ser Asn Thr Glu Val Phe Leu Leu Phe Ser Asn Phe
        755                 760                 765

Asp Asn Gly Lys Arg Pro Ser Thr Leu His Gln Met Asn Thr Lys Leu
    770                 775                 780

Ser Ala Val Tyr Ala Gly Glu Ala Met His Thr Ala Gly Cys
785                 790                 795

<210>  3
<211>  482
<212>  PRT
<213>  Artificial Sequence

<400>  3

Ala Pro Ser Tyr Arg Val Lys Arg Ala Asp Ile Ala Thr Cys Thr Glu
1               5                   10                  15

Ala Ala Val Val Asn Ala Ala Asn Ala Arg Gly Thr Val Gly Asp Gly
            20                  25                  30

Val Cys Arg Ala Val Ala Lys Lys Trp Pro Ser Ala Phe Lys Gly Ala

        35                  40                  45

Ala Thr Pro Val Gly Thr Ile Lys Thr Val Met Cys Gly Ser Tyr Pro
    50                  55                  60

Val Ile His Ala Val Ala Pro Asn Phe Ser Ala Thr Thr Glu Ala Glu
65                  70                  75                  80

Gly Asp Arg Glu Leu Ala Ala Val Tyr Arg Ala Val Ala Ala Glu Val
                85                  90                  95

Asn Arg Leu Ser Leu Ser Ser Val Ala Ile Pro Leu Leu Ser Thr Gly
            100                 105                 110

Val Phe Ser Gly Gly Arg Asp Arg Leu Gln Gln Ser Leu Asn His Leu
        115                 120                 125

Phe Thr Ala Met Asp Ala Thr Asp Ala Asp Val Thr Ile Tyr Cys Arg
    130                 135                 140

Asp Lys Ser Trp Glu Lys Lys Ile Gln Glu Ala Ile Asp Met Arg Thr
145                 150                 155                 160

Ala Val Glu Leu Leu Asn Asp Asp Val Glu Leu Thr Thr Asp Leu Val
                165                 170                 175

Arg Val His Pro Asp Ser Ser Leu Val Gly Arg Lys Gly Tyr Ser Thr
            180                 185                 190

Thr Asp Gly Ser Leu Tyr Ser Tyr Phe Glu Gly Thr Lys Phe Asn Gln
        195                 200                 205

Ala Ala Ile Asp Met Ala Glu Ile Leu Thr Leu Trp Pro Arg Leu Gln
    210                 215                 220

Glu Ala Asn Glu Arg Ile Cys Leu Tyr Ala Leu Gly Glu Thr Met Asp
225                 230                 235                 240

Asn Ile Gly Ser Lys Cys Pro Val Asn Asp Ser Asp Ser Ser Thr Pro
                245                 250                 255

Pro Arg Thr Val Pro Cys Leu Cys Arg Tyr Ala Met Thr Ala Glu Arg
            260                 265                 270

Ile Ala Arg Leu Arg Ser His Gln Val Lys Ser Met Val Val Cys Ser
        275                 280                 285

Ser Phe Pro Leu Pro Lys Tyr His Val Asp Gly Val Gln Lys Val Lys
    290                 295                 300

Cys Glu Lys Val Leu Leu Phe Asp Pro Thr Val Pro Ser Val Val Ser
305                 310                 315                 320

Pro Arg Lys Tyr Ala Ala Ser Thr Thr Asp His Ser Asp Arg Ser Leu
                325                 330                 335

Arg Gly Phe Asp Leu Asp Trp Thr Thr Asp Ser Ser Ser Thr Ala Ser
            340                 345                 350

Asp Thr Met Ser Leu Pro Ser Leu Gln Ser Cys Asp Ile Asp Ser Ile
        355                 360                 365

Tyr Glu Pro Met Ala Pro Ile Val Val Thr Ala Asp Val His Pro Glu
    370                 375                 380

Pro Ala Gly Ile Ala Asp Leu Ala Ala Asp Val His Pro Glu Pro Ala
385                 390                 395                 400

Asp His Val Asp Leu Glu Asn Pro Ile Pro Pro Pro Arg Pro Lys Arg
                405                 410                 415

Ala Ala Tyr Leu Ala Ser Arg Ala Ala Glu Arg Pro Val Pro Ala Pro
            420                 425                 430

Arg Lys Pro Thr Pro Ala Pro Arg Thr Ala Phe Arg Asn Lys Leu Pro
        435                 440                 445

Leu Thr Phe Gly Asp Phe Asp Glu His Glu Val Asp Ala Leu Ala Ser
    450                 455                 460

Gly Ile Thr Phe Gly Asp Phe Asp Asp Val Leu Arg Leu Gly Arg Ala
465                 470                 475                 480

Gly Ala

<210>  4
<211>  614
<212>  PRT
<213>  Artificial Sequence

<400>  4

Tyr Ile Phe Ser Ser Asp Thr Gly Ser Gly His Leu Gln Gln Lys Ser
1               5                   10                  15

Val Arg Gln His Asn Leu Gln Cys Ala Gln Leu Asp Ala Val Gln Glu
            20                  25                  30

Glu Lys Met Tyr Pro Pro Lys Leu Asp Thr Glu Arg Glu Lys Leu Leu
        35                  40                  45

Leu Leu Lys Met Gln Met His Pro Ser Glu Ala Asn Lys Ser Arg Tyr
    50                  55                  60

Gln Ser Arg Lys Val Glu Asn Met Lys Ala Thr Val Val Asp Arg Leu
65                  70                  75                  80

Thr Ser Gly Ala Arg Leu Tyr Thr Gly Ala Asp Val Gly Arg Ile Pro
                85                  90                  95

Thr Tyr Ala Val Arg Tyr Pro Arg Pro Val Tyr Ser Pro Thr Val Ile
            100                 105                 110

Glu Arg Phe Ser Ser Pro Asp Val Ala Ile Ala Ala Cys Asn Glu Tyr
        115                 120                 125

Leu Ser Arg Asn Tyr Pro Thr Val Ala Ser Tyr Gln Ile Thr Asp Glu
    130                 135                 140

Tyr Asp Ala Tyr Leu Asp Met Val Asp Gly Ser Asp Ser Cys Leu Asp
145                 150                 155                 160

Arg Ala Thr Phe Cys Pro Ala Lys Leu Arg Cys Tyr Pro Lys His His
                165                 170                 175

Ala Tyr His Gln Pro Thr Val Arg Ser Ala Val Pro Ser Pro Phe Gln
            180                 185                 190

Asn Thr Leu Gln Asn Val Leu Ala Ala Ala Thr Lys Arg Asn Cys Asn
        195                 200                 205

Val Thr Gln Met Arg Glu Leu Pro Thr Met Asp Ser Ala Val Phe Asn
    210                 215                 220

Val Glu Cys Phe Lys Arg Tyr Ala Cys Ser Gly Glu Tyr Trp Glu Glu
225                 230                 235                 240

Tyr Ala Lys Gln Pro Ile Arg Ile Thr Thr Glu Asn Ile Thr Thr Tyr
                245                 250                 255

Val Thr Lys Leu Lys Gly Pro Lys Ala Ala Ala Leu Phe Ala Lys Thr
            260                 265                 270

His Asn Leu Val Pro Leu Gln Glu Val Pro Met Asp Arg Phe Thr Val
        275                 280                 285

Asp Met Lys Arg Asp Val Lys Val Thr Pro Gly Thr Lys His Thr Glu
    290                 295                 300

Glu Arg Pro Lys Val Gln Val Ile Gln Ala Ala Glu Pro Leu Ala Thr
305                 310                 315                 320

Ala Tyr Leu Cys Gly Ile His Arg Glu Leu Val Arg Arg Leu Asn Ala
                325                 330                 335

Val Leu Arg Pro Asn Val His Thr Leu Phe Asp Met Ser Ala Glu Asp
            340                 345                 350

Phe Asp Ala Ile Ile Ala Ser His Phe His Pro Gly Asp Pro Val Leu
        355                 360                 365

Glu Thr Asp Ile Ala Ser Phe Asp Lys Ser Gln Asp Asp Ser Leu Ala
    370                 375                 380

Leu Thr Gly Leu Met Ile Leu Glu Asp Leu Gly Val Asp Gln Tyr Leu
385                 390                 395                 400

Leu Asp Leu Ile Glu Ala Ala Phe Gly Glu Ile Ser Ser Cys His Leu
                405                 410                 415

Pro Thr Gly Thr Arg Phe Lys Phe Gly Ala Met Met Lys Ser Gly Met
            420                 425                 430

Phe Leu Thr Leu Phe Ile Asn Thr Val Leu Asn Ile Thr Ile Ala Ser
        435                 440                 445

Arg Val Leu Glu Gln Arg Leu Thr Asp Ser Ala Cys Ala Ala Phe Ile
    450                 455                 460

Gly Asp Asp Asn Ile Val His Gly Val Ile Ser Asp Lys Leu Met Ala
465                 470                 475                 480

Glu Arg Cys Ala Ser Trp Val Asn Met Glu Val Lys Ile Ile Asp Ala
                485                 490                 495

Val Met Gly Glu Lys Pro Pro Tyr Phe Cys Gly Gly Phe Ile Val Phe
            500                 505                 510

Asp Ser Val Thr Gln Thr Ala Cys Arg Val Ser Asp Pro Leu Lys Arg
        515                 520                 525

Leu Phe Lys Leu Gly Lys Pro Leu Thr Ala Glu Asp Lys Gln Asp Glu
    530                 535                 540

Asp Arg Arg Arg Ala Leu Ser Asp Glu Val Ser Lys Trp Phe Arg Thr
545                 550                 555                 560

Gly Leu Gly Ala Glu Leu Glu Val Ala Leu Thr Ser Arg Tyr Glu Val
                565                 570                 575

Glu Gly Cys Lys Ser Ile Leu Ile Ala Met Thr Thr Leu Ala Arg Asp
            580                 585                 590

Ile Lys Ala Phe Lys Lys Leu Arg Gly Pro Val Ile His Leu Tyr Gly
        595                 600                 605

Gly Pro Arg Leu Val Arg
    610

<210>  5
<211>  193
<212>  DNA
<213>  Artificial Sequence

<400>  5
gagtggacct gtgtcgcgta aatctaaaag taagagtgag gcagaatctt tttcggatag      60

tggcgcttct gagccactaa gttcataatc aagatgtctt actctacttc tggtttgcgt     120

tctttgcctg catatactaa gtctttttgt ccttattatg ctttgtatga tctgttggtg     180

tcagcccaag gtg                                                        193

<210>  6
<211>  1614
<212>  DNA
<213>  Artificial Sequence

<400>  6
atggccgcca aagtgcatgt tgatattgag gctgacagcc cattcatcaa gtctttgcag      60

aaggcatttc cgtcgttcga ggtggagtca ttgcaggtca caccaaatga ccatgcaaat     120

gccagagcat tttcgcacct ggctaccaaa ttgatcgagc aggagactga caaagacaca     180

ctcatcttgg atatcggcag tgcgccttcc aggagaatga tgtctacgca caaataccac     240

tgcgtatgcc ctatgcgcag cgcagaagac cccgaaaggc tcgatagcta cgcaaagaaa     300

ctggcagcgg cctccgggaa ggtgctggat agagagatcg caggaaaaat caccgacctg     360

cagaccgtca tggctacgcc agacgctgaa tctcctacct tttgcctgca tacagacgtc     420

acgtgtcgta cggcagccga agtggccgta taccaggacg tgtatgctgt acatgcacca     480

acatcgctgt accatcaggc gatgaaaggt gtcagaacgg cgtattggat tgggtttgac 540

accaccccgt ttatgtttga cgcgctagca ggcgcgtatc caacctacgc cacaaactgg 600

gccgacgagc aggtgttaca ggccaggaac ataggactgt gtgcagcatc cttgactgag 660

ggaagactcg gcaaactgtc cattctccgc aagaagcaat tgaaaccttg cgacacagtc 720

atgttctcgg taggatctac attgtacact gagagcagaa agctactgag gagctggcac 780

ttaccctccg tattccacct gaaaggtaaa caatccttta cctgtaggtg cgataccatc 840

gtatcatgtg aagggtacgt agttaagaaa atcactatgt gccccggcct gtacggtaaa 900

acggtagggt acgccgtgac gtatcacgcg gagggattcc tagtgtgcaa gaccacagac 960

actgtcaaag gagaaagagt ctcattccct gtatgcacct acgtcccctc aaccatctgt 1020

gatcaaatga ctggcatact agcgaccgac gtcacaccgg aggacgcaca gaagttgtta 1080

gtgggattga atcagaggat agttgtgaac ggaagaacac agcgaaacac taacacgatg 1140

aagaactatc tgcttccgat tgtggccgtc gcatttagca agtgggcgag ggaatacaag 1200

gcagaccttg atgatgaaaa acctctgggt gtccgagaga ggtcacttac ttgctgctgc 1260

ttgtgggcat ttaaaacgag gaagatgcac accatgtaca agaaaccaga cacccagaca 1320

atagtgaagg tgccttcaga gtttaactcg ttcgtcatcc cgagcctatg gtctacaggc 1380

ctcgcaatcc cagtcagatc acgcattaag atgcttttgg ccaagaagac caagcgagag 1440

ttaatacctg ttctcgacgc gtcgtcagcc agggatgctg aacaagagga gaaggagagg 1500

ttggaggccg agctgactag agaagcctta ccacccctcg tccccatcgc gccggcggag 1560

acgggagtcg tcgacgtcga cgttgaagaa ctagagtatc acgcaggtgc ataa 1614

<210> 7
<211> 2400
<212> DNA
<213> Artificial Sequence

<400> 7
atgggggtcg tggaaacacc tcgcagcgcg ttgaaagtca ccgcacagcc gaacgacgta 60

ctactaggaa attacgtagt tctgtccccg cagaccgtgc tcaagagctc caagttggcc 120

cccgtgcacc ctctagcaga gcaggtgaaa ataataacac ataacgggag ggccggcggt 180

taccaggtcg acggatatga cggcagggtc ctactaccat gtggatcggc cattccggtc 240

cctgagtttc aagctttgag cgagagcgcc actatggtgt acaacgaaag ggagttcgtc 300

aacaggaaac tataccatat tgccgttcac ggaccgtcgc tgaacaccga cgaggagaac 360

tacgagaaag tcagagctga aagaactgac gccgagtacg tgttcgacgt agataaaaaa 420

tgctgcgtca agagagagga agcgtcgggt ttggtgttgg tgggagagct aaccaacccc 480

ccgttccatg aattcgccta cgaagggctg aagatcaggc cgtcggcacc atataagact 540

acagtagtag gagtctttgg ggttccggga tcaggcaagt ctgctattat taagagcctc 600

gtgaccaaac acgatctggt caccagcggc aagaaggaga actgccagga aatagttaac 660

gacgtgaaga agcaccgcgg gaaggggaca agtagggaaa acagtgactc catcctgcta 720

aacgggtgtc gtcgtgccgt ggacatccta tatgtggacg aggctttcgc ttgccattcc 780

ggtactctgc tggccctaat tgctcttgtt aaacctcgga gcaaagtggt gttatgcgga 840

gaccccaagc aatgcggatt cttcaatatg atgcagctta aggtgaactt caaccacaac 900

atctgcactg aagtatgtca taaaagtata tccagacgtt gcacgcgtcc agtcacggcc 960

atcgtgtcta cgttgcacta cggaggcaag atgcgcacga ccaacccgtg caacaaaccc 1020

ataatcatag acaccacagg acagaccaag cccaagccag gagacatcgt gttaacatgc 1080

ttccgaggct gggcaaagca gctgcagttg gactaccgtg gacacgaagt catgacagca 1140

gcagcatctc agggcctcac ccgcaaaggg gtatacgccg taaggcagaa ggtgaatgaa 1200

aatcccttgt atgcccctgc gtcggagcac gtgaatgtac tgctgacgcg cactgaggat 1260

aggctggtgt ggaaaacgct ggccggcgat ccctggatta aggtcctatc aaacattcca 1320

cagggtaact ttacggccac attggaagaa tggcaagaag aacacgacaa aataatgaag 1380

gtgattgaag gaccggctgc gcctgtggac gcgttccaga acaaagcgaa cgtgtgttgg 1440

gcgaaaagcc tggtgcctgt cctggacact gccggaatca gattgacagc agaggagtgg 1500

agcaccataa ttacagcatt taaggaggac agagcttact ctccagtggt ggccttgaat 1560

gaaatttgca ccaagtacta tggagttgac ctggacagtg gcctgttttc tgccccgaag 1620

gtgtccctgt attacgagaa caaccactgg gataacagac ctggtggaag gatgtatgga 1680

ttcaatgccg caacagctgc caggctggaa gctagacata ccttcctgaa ggggcagtgg 1740

catacgggca agcaggcagt tatcgcagaa agaaaaatcc aaccgctttc tgtgctggac 1800

aatgtaattc ctatcaaccg caggctgccg cacgccctgg tggctgagta caagacggtt 1860

aaaggcagta gggttgagtg gctggtcaat aaagtaagag ggtaccacgt cctgctggtg 1920

agtgagtaca acctggcttt gcctcgacgc agggtcactt ggttgtcacc gctgaatgtc 1980

acaggcgccg ataggtgcta cgacctaagt ttaggactgc cggctgacgc cggcaggttc 2040

gacttggtct ttgtgaacat tcacacggaa ttcagaatcc accactacca gcagtgtgtc 2100

gaccacgcca tgaagctgca gatgcttggg ggagatgcgc tacgactgct aaaacccggc 2160

ggcatcttga tgagagctta cggatacgcc gataaaatca gcgaagccgt tgtttcctcc 2220

ttaagcagaa agttctcgtc tgcaagagtg ttgcgcccgg attgtgtcac cagcaataca 2280

gaagtgttct tgctgttctc caactttgac aacggaaaga gaccctctac gctacaccag 2340

atgaatacca agctgagtgc cgtgtatgcc ggagaagcca tgcacacggc cgggtgttaa 2400

<210>  8
<211>  1452
<212>  DNA
<213>  Artificial Sequence

<400>  8
atggcaccat cctacagagt taagagagca gacatagcca cgtgcacaga agcggctgtg      60

gttaacgcag ctaacgcccg tggaactgta ggggatggcg tatgcagggc cgtggcgaag     120

aaatggccgt cagcctttaa gggagcagca acaccagtgg gcacaattaa aacagtcatg     180

tgcggctcgt accccgtcat ccacgctgta gcgcctaatt tctctgccac gactgaagcg     240

gaaggggacc gcgaattggc cgctgtctac cgggcagtgg ccgccgaagt aaacagactg     300

tcactgagca gcgtagccat cccgctgctg tccacaggag tgttcagcgg cggaagagat     360

aggctgcagc aatccctcaa ccatctattc acagcaatgg acgccacgga cgctgacgtg     420

accatctact gcagagacaa aagttgggag aagaaaatcc aggaagccat tgacatgagg     480

acggctgtgg agttgctcaa tgatgacgtg gagctgacca cagacttggt gagagtgcac     540

ccggacagca gcctggtggg tcgtaagggc tacagtacca ctgacgggtc gctgtactcg     600

tactttgaag gtacgaaatt caaccaggct gctattgata tggcagagat actgacgttg     660

tggcccagac tgcaggaggc aaacgaacgg atatgcctat acgcgctggg cgaaacaatg     720

gacaacatcg gatccaaatg tccggtgaac gattccgatt catcaacacc tcccaggaca     780

gtgccctgcc tgtgccgcta cgcaatgaca gcagaacgga tcgcccgcct taggtcacac     840

caagttaaaa gcatggtggt ttgctcatct ttccccctcc cgaaatacca tgtagatggg     900

gtgcagaagg taaagtgcga gaaggttctc ctgttcgacc cgacggtacc ttcagtggtt     960

agtccgcgga agtatgccgc atctacgacg gaccactcag atcggtcgtt acgagggttt    1020

gacttggact ggaccaccga ctcgtcttcc actgccagcg ataccatgtc gctacccagt    1080

ttgcagtcgt gtgacatcga ctcgatctac gagccaatgg ctcccatagt agtgacggct    1140

gacgtacacc ctgaacccgc aggcatcgcg gacctggcgg cagatgtgca ccctgaaccc    1200

gcagaccatg tggacctcga gaacccgatt cctccaccgc gcccgaagag agctgcatac    1260

cttgcctccc gcgcggcgga gcgaccggtg ccggcgccga gaaagccgac gcctgcccca    1320

aggactgcgt ttaggaacaa gctgcctttg acgttcggcg actttgacga gcacgaggtc    1380

gatgcgttgg cctccgggat tactttcgga gacttcgacg acgtcctgcg actaggccgc    1440

gcgggtgcat aa                                                        1452

<210>  9
<211>  1908
<212>  DNA
<213>  Artificial Sequence

<400>  9

atgtatattt tctcctcgga cactggcagc ggacatttac aacaaaaatc cgttaggcag 60

cacaatctcc agtgcgcaca actggatgcg gtccaggagg agaaaatgta cccgccaaaa 120

ttggatactg agagggagaa gctgttgctg ctgaaaatgc agatgcaccc atcggaggct 180

aataagagtc gataccagtc tcgcaaagtg gagaacatga aagccacggt ggtggacagg 240

ctcacatcgg gggccagatt gtacacggga gcggacgtag gccgcatacc aacatacgcg 300

gttcggtacc cccgccccgt gtactcccct accgtgatcg aaagattctc aagccccgat 360

gtagcaatcg cagcgtgcaa cgaataccta tccagaaatt acccaacagt ggcgtcgtac 420

cagataacag atgaatacga cgcatacttg gacatggttg acgggtcgga tagttgcttg 480

gacagagcga cattctgccc ggcgaagctc cggtgctacc cgaaacatca tgcgtaccac 540

cagccgactg tacgcagtgc cgtcccgtca ccctttcaga acacactaca gaacgtgcta 600

gcggccgcca ccaagagaaa ctgcaacgtc acgcaaatgc gagaactacc caccatggac 660

tcggcagtgt tcaacgtgga gtgcttcaag cgctatgcct gctccggaga atattgggaa 720

gaatatgcta aacaacctat ccggataacc actgagaaca tcactaccta tgtgaccaaa 780

ttgaaaggcc cgaaagctgc tgccttgttc gctaagaccc acaacttggt tccgctgcag 840

gaggttccca tggacagatt cacggtcgac atgaaacgag atgtcaaagt cactccaggg 900

acgaaacaca cagaggaaag acccaaagtc caggtaattc aagcagcgga gccattggcg 960

accgcttacc tgtgcggcat ccacagggaa ttagtaagga gactaaatgc tgtgttacgc 1020

cctaacgtgc acacattgtt tgatatgtcg gccgaagact ttgacgcgat catcgcctct 1080

cacttccacc caggagaccc ggttctagag acggacattg catcattcga caaaagccag 1140

gacgactcct tggctcttac aggtttaatg atcctcgaag atctaggggt ggatcagtac 1200

ctgctggact tgatcgaggc agcctttggg gaaatatcca gctgtcacct accaactggc 1260

acgcgcttca agttcggagc tatgatgaaa tcgggcatgt ttctgacttt gtttattaac 1320

actgttttga acatcaccat agcaagcagg gtactggagc agagactcac tgactccgcc 1380

tgtgcggcct tcatcggcga cgacaacatc gttcacggag tgatctccga caagctgatg 1440

gcggagaggt gcgcgtcgtg ggtcaacatg gaggtgaaga tcattgacgc tgtcatgggc 1500

gaaaaacccc catatttttg tgggggattc atagtttttg acagcgtcac acagaccgcc 1560

tgccgtgttt cagacccact taagcgcctg ttcaagttgg gtaagccgct aacagctgaa 1620

gacaagcagg acgaagacag gcgacgagca ctgagtgacg aggttagcaa gtggttccgg 1680

acaggcttgg gggccgaact ggaggtggca ctaacatcta ggtatgaggt agagggctgc 1740

aaaagtatcc tcatagccat gaccaccttg gcgaggacat ttaaggcgtt taagaaattg 1800

agaggacctg ttatacacct ctacggcggt cctagattgg tgcgttaata cacagaattc 1860

tgattatagc gcactattat agcaccatga attacatccc tacgctaa 1908